# EUROPEAN PATENT APPLICATION

(11) **EP 4 074 692 A1**
(43) Date of publication of application: **19.10.2022**
(21) Application number: 20898053.2
(22) Date of filing: 08.12.2020
(51) Int. Cl.: C07C 259/10, C07F 1/08, C07F 3/02, C07F 3/06, C07F 15/04, C07F 15/06, F17C 11/00, C01B 3/00, C07D 213/60, C07D 213/81, C07D 487/04, B01J 20/26

(54) **GAS-STORING MATERIAL INCLUDING METAL/ORGANIC FRAMEWORK BONDED BY HYDROXAMIC ACID**

(30) Priority: 11.12.2019 JP 2019223660; 26.02.2020 JP 2020030761
(71) Applicant: Rikkyo Educational Corporation, Tokyo 171-8501 (JP); Nippon Soda Co., Ltd., Tokyo 100-8165 (JP)
(72) Inventor: MINOURA, Mao, Tokyo 171-8501 (JP); SUGAMATA, Koh, Tokyo 171-8501 (JP); IIHAMA, Teruyuki, Tokyo 100-8165 (JP)
(74) Representative: Wibbelmann, Jobst
(86) International application number: PCT/JP2020/045734
(87) International publication number: WO 2021/117741

(57) **Abstract**

It is an object of the present invention to provide a storage material for a gas such as hydrogen, carbon dioxide, methane, or acetylene (excluding nitrogen gas) comprising a metal-organic framework comprising a hydroxamic acid group as a bonding site. A metal-organic framework comprising a multivalent metal ion and a molecule comprising an unsubstituted or substituted hydroxamic acid group and one or more sites capable of being bonded to the multivalent metal ion is contained. The molecule is preferably at least one compound selected from the group consisting of compounds of formulas (I) to (III).

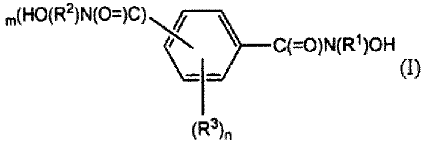

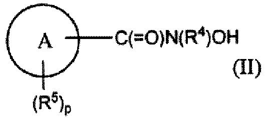

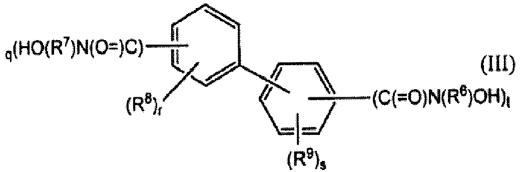

## Description

### Technical Field

The present invention relates to a novel storage material for a gas other than nitrogen containing a metal-organic framework bonded by a hydroxamic acid, and a method for storing a gas other than nitrogen and a storage tank for a gas other than nitrogen using such a storage material for a gas other than nitrogen. This application claims priority to Japanese Patent Application No. 2019-223660 filed on December 11, 2019, and Japanese Patent Application No. 2020-030761 filed on February 26, 2020, the contents of which are incorporated herein.

### Background Art

A metal-organic framework (hereinafter sometimes referred to as a "MOF") is a substance in the form of a solid having a polymer structure having spaces (that is, pores) inside by combining metal ions with crosslinkable organic ligands linking them, and has attracted high interest as a porous material having functions such as gas storage and separation for these dozen or so years. Much research on using terephthalic acid as an organic ligand is done, and it is known that MOF-5 obtained by a solvothermal method using Zn(NO₃)₂•6H₂O in DMF, using terephthalic acid as a crosslinkable organic ligand can store 7.1% by mass of hydrogen based on MOF-5 under the conditions of a temperature of 77 K and 4 MPa (see patent document 1 and non-patent documents 1 to 3).

Meanwhile, hydroxamic acids (-CONHOH), as well as carboxylic acids (-COOH), are known to be strongly coordinated to metals. However, for metal-organic frameworks using as a ligand 1,4-benzene-dicarbohydroxamic acid in which the carboxylic acid sites of terephthalic acid are replaced by hydroxamic acids, only two examples have been reported so far.

It is reported that by heating UiO-66, which is a MOF obtained by a solvothermal method using zirconium tetrachloride in DMF, using terephthalic acid as an organic ligand, and 1,4-benzene-dicarbohydroxamic acid (H2BDHA) in DMF, the terephthalate forming UiO-66 is replaced by 1,4-benzene-dicarbohydroxamic acid to form UiO-66-H2BDHA (non-patent document 4).

It is reported that MUV-11, which is a MOF having a hydroxamic acid site, is obtained by a solvothermal method in which 1,4-benzene-dicarbohydroxamic acid is heated to 120°C in DMF together with tetraisopropyl orthotitanate and acetic acid (non-patent document 5).

### Prior Art Documents

### Patent Documents

Patent document 1: U.S. Patent Application Publication No. 2010-75123

### Non-patent Documents

Non-patent document 1: H. Li, M. Eddaudi, M. O'Keefe, O. M. Yaghi, Nature, 402, 276(1999)
Non-patent document 2: M. Eddaudi, J. Kim, N. Rosi, D. Vodak, J. Wachter, M. O'Keefe, O. M. Yaghi, Science 2002, 295(5554), 469.
Non-patent document 3: S. Kaye, A. Daily, O. M. Yaghi, J. Long, J. Am. Chem. Soc. 2007, 129(46), 14176.
Non-patent document 4: C. Pereira, A. Howarth, N. Vermeulen, F. Almeida Paz, J. Tome, J. Hupp, O. Farha, Mater. Chem. Front. 2017, 1, 1194.
Non-patent document 5: N. Padial, J. Castells-Gil, N. Almora-Barrios, M. Romero-Angel, I. Silva, M. Barawi, A. Garcia-Sanchez, V. O'Shea, C. Marti-Gastaldo, J. Am. Chem. Soc. 2019, 141, 13124.

### Summary of the Invention

### Object to be Solved by the Invention

The structures of MOFs are known to change greatly depending on the metal species, ligand, and reaction conditions used. Regarding MOFs comprising hydroxamic acids as bonding sites, there are few reported examples, and their ability to store a gas other than nitrogen is also unknown.

It is an object of the present invention to provide a storage material for a gas other than nitrogen containing a MOF comprising a hydroxamic acid group as a bonding site.

### Means to Solve the Object

The present inventors have studied diligently in order to solve the above problems, and as a result, found that a MOF is obtained even with a combination of a metal species and a molecule having a hydroxamic acid as a bonding site, other than those of the prior art documents, and moreover the MOF has the ability to store a gas other than nitrogen. Thus, the present inventors have completed the present invention.

That is, the present invention is the following specified by the items shown below.
[1] A storage material for a gas other than nitrogen, comprising a metal-organic framework wherein a multivalent metal ion is bonded to a molecule comprising an unsubstituted or substituted hydroxamic acid group and one or more sites capable of being bonded to the multivalent metal ion.
[2] The storage material for a gas other than nitrogen according to [1], wherein the site capable of being bonded to the multivalent metal ion is a nitrogen atom in the unsubstituted or substituted hydroxamic acid group or a nitrogen-containing heterocyclic group.
[3] The storage material for a gas other than nitrogen according to [1] or [2], wherein the molecule comprising an unsubstituted or substituted hydroxamic acid group and one or more sites capable of being bonded to the multivalent metal ion is at least one compound selected from a group of compounds of the following formulas (I) to (III): 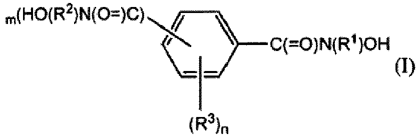 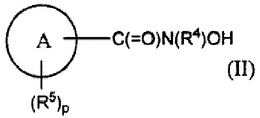 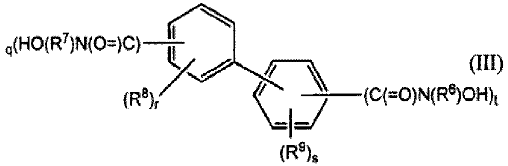 (in formulas (I) to (III),
   R¹, R², R⁴, R⁶, and R⁷ each independently are a hydrogen atom, a C1-6 alkyl group, a C6-10 aryl group, a C1-6 alkylcarbonyl group, or a C6-10 arylcarbonyl group and optionally form a ring together with a carbon adjacent to a carbon of a ring to which C(=O)N(R^{u})OH (u = 1, 2, 4, 6, or 7) is bonded,
   R³, R⁵, R⁸, and R⁹ each independently are a C1-6 alkyl group, a C3-8 cycloalkyl group, a C6-10 aryl group, a three-to six-membered heterocyclyl group, a C1-6 alkoxy group, a C6-10 aryloxy group, a heteroaryloxy group, a halogeno group, a C1-6 haloalkyl group, a C6-10 haloaryl group, a C1-6 haloalkoxy group, a C1-6 alkylthio group, a C6-10 arylthio group, a heteroarylthio group, a C1-6 alkylsulfinyl group, a C6-10 arylsulfinyl group, a heteroarylsulfinyl group, a C1-6 alkylsulfonyl group, a C6-10 arylsulfonyl group, a heteroarylsulfonyl group, a cyano group, a nitro group, or a group represented by NR¹¹R¹² (wherein R¹¹ and R¹² each independently are a hydrogen atom, a C1-6 alkyl group, a C6-10 aryl group, a C1-6 alkylcarbonyl group, or a C6-10 arylcarbonyl group),
   m is the number of groups represented by C(=O)N(R²)OH and is 1 or 2, and when m is 2, R² is the same or different from each other,
   q is the number of groups represented by C(=O)N(R⁷)OH and is 1 or 2, and when q is 2, R⁷ is the same or different from each other,
   t is the number of groups represented by C(=O)N(R⁶)OH and is 1 or 2, and when t is 2, R⁶ is the same or different from each other,
   n is the number of R³ and is an integer of 0 or any of 1 to 4, and when n is 2 or more, R³ is the same or different from each other,
   p is the number of R⁵, and p is an integer of 0 or any of 1 to 3 when A is a five-membered ring, and is an integer of 0 or any of 1 to 4 when A is a six-membered ring, and when p is 2 or more, R⁵ is the same or different from each other,
   r is the number of R⁸ and is an integer of 0 or any of 1 to 4, and when r is 2 or more, R⁸ is the same or different,
   s is the number of R⁹ and is an integer of 0 or any of 1 to 4, and when s is 2 or more, R⁹ is the same or different,
   provided that n + m ≤ 5, r + q ≤ 5, and s + t ≤ 5, and
   in formula (II), A is a five- or six-membered aromatic heterocycle containing 1 to 4 nitrogen atoms in an integer number as a ring-constituting atom.)
[4] The storage material for a gas other than nitrogen according to any one of [1] to [3], wherein the multivalent metal ion is an ion of at least one metal selected from the group consisting of metals of groups 2 to 13 of the periodic table of elements.
[5] The storage material for a gas other than nitrogen according to any one of [1] to [4], wherein the multivalent metal ion is an ion of at least one metal selected from Zn, Fe, Co, Ni, Cu, Al, Zr, and Mg.
[6] A method for storing a gas other than nitrogen, comprising a step of bringing a gas other than nitrogen into contact with the storage material for a gas other than nitrogen according to any one of [1] to [5] to adsorb or occlude the gas inside the storage material for a gas other than nitrogen.
[7] A storage tank for a gas other than nitrogen, filled with the storage material for a gas other than nitrogen according to any one of [1] to [5].
[8] A metal-organic framework wherein a multivalent metal ion is bonded to a molecule comprising an unsubstituted or substituted hydroxamic acid group and one or more sites capable of being bonded to the multivalent metal ion (provided that a metal-organic framework formed by a bond between Ti⁴⁺ or Zr⁴⁺ and benzene-1,4-dicarbohydroxamic acid is excluded).
[9] The metal-organic framework according to [8], wherein the molecule comprising an unsubstituted or substituted hydroxamic acid group and one or more sites capable of being bonded to the multivalent metal ion is at least one compound selected from a group of compounds of the following formulas (I) to (III): 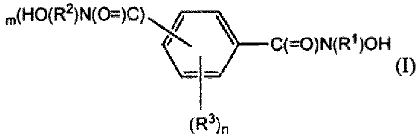 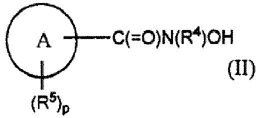 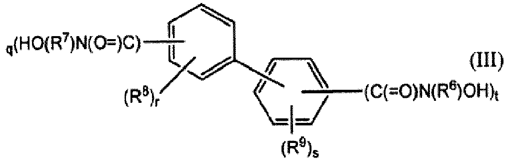 (in formulas (I) to (III),
   R¹, R², R⁴, R⁶, and R⁷ each independently are a hydrogen atom, a C1-6 alkyl group, a C6-10 aryl group, a C1-6 alkylcarbonyl group, or a C6-10 arylcarbonyl group and optionally form a ring together with a carbon adjacent to a carbon of a ring to which C(=O)N(R^{u})OH (u = 1, 2, 4, 6, or 7) is bonded,
   R³, R⁵, R⁸, and R⁹ each independently are a C1-6 alkyl group, a C3-8 cycloalkyl group, a C6-10 aryl group, a three-to six-membered heterocyclyl group, a C1-6 alkoxy group, a C6-10 aryloxy group, a heteroaryloxy group, a halogeno group, a C1-6 haloalkyl group, a C6-10 haloaryl group, a C1-6 haloalkoxy group, a C1-6 alkylthio group, a C6-10 arylthio group, a heteroarylthio group, a C1-6 alkylsulfinyl group, a C6-10 arylsulfinyl group, a heteroarylsulfinyl group, a C1-6 alkylsulfonyl group, a C6-10 arylsulfonyl group, a heteroarylsulfonyl group, a cyano group, a nitro group, or a group represented by NR¹¹R¹² (wherein R¹¹ and R¹² each independently are a hydrogen atom, a C1-6 alkyl group, a C6-10 aryl group, a C1-6 alkylcarbonyl group, or a C6-10 arylcarbonyl group),
   m is the number of groups represented by C(=O)N(R²)OH and is 1 or 2, and when m is 2, R² is the same or different from each other,
   q is the number of groups represented by C(=O)N(R⁷)OH and is 1 or 2, and when q is 2, R⁷ is the same or different from each other,
   t is the number of groups represented by C(=O)N(R⁶)OH and is 1 or 2, and when t is 2, R⁶ is the same or different from each other,
   n is the number of R³ and is an integer of 0 or any of 1 to 4, and when n is 2 or more, R³ is the same or different from each other,
   p is the number of R⁵, and p is an integer of 0 or any of 1 to 3 when A is a five-membered ring, and p is an integer of 0 or any of 1 to 4 when A is a six-membered ring, and when p is 2 or more, R⁵ is the same or different from each other,
   r is the number of R⁸ and is an integer of 0 or any of 1 to 4, and when r is 2 or more, R⁸ is the same or different,
   s is the number of R⁹ and is an integer of 0 or any of 1 to 4, and when s is 2 or more, R⁹ is the same or different,
   provided that n + m ≤ 5, r + q ≤ 5, and s + t ≤ 5, and
   in formula (II), A is a five- or six-membered aromatic heterocycle containing 1 to 4 nitrogen atoms in an integer number as a ring-constituting atom.)
[10] The metal-organic framework according to [8] or [9], wherein the multivalent metal ion is an ion of at least one metal selected from the group consisting of metals of groups 2 to 13 of the periodic table of elements.
[11] The metal-organic framework according to any one of [8] to [10], wherein the multivalent metal ion is an ion of at least one metal selected from Zn, Fe, Co, Ni, Cu, Al, Zr, and Mg.

### Effect of the Invention

The storage material for a gas other than nitrogen according to the present invention is novel, and by using this material, a gas such as hydrogen, carbon dioxide, methane, or acetylene (excluding nitrogen) can be stored.

### Mode of Carrying Out the Invention

A storage material for a gas other than nitrogen according to the present invention comprises a metal-organic framework wherein a multivalent metal ion is bonded to a molecule comprising an unsubstituted or substituted hydroxamic acid group and one or more sites capable of being bonded to the multivalent metal ion (hereinafter referred to as a "hydroxamic acid group-containing molecule").

The multivalent metal ion used in the present invention is not particularly limited as long as it is an ion of a divalent or higher-valent metal. The multivalent metal ion is preferably an ion of at least one metal selected from the group consisting of the metals of groups 2 to 13 of the periodic table of elements, further preferably an ion of at least one metal selected from Zn, Fe, Co, Ni, Cu, Al, Zr, and Mg, and further preferably an ion of at least one metal selected from Co, Ni, Cu, and Zn. One of these can be used singly, or two or more of these can be mixed and used.

These multivalent metal ions are supplied in the form of various salts, but considering the purity of the salt, the ease of bonding between the metal ion and the hydroxamic acid group-containing molecule, and the like, a nitrate is preferable, and specifically, Zn(NO₃)₂•6H₂O, Zn(NO₃)₂•4H₂O, Ni(NO₃)₂•6H₂O, Mg(NO₃)₂•6H₂O, Cu(NO₃)₂•H₂O, Cu(NO₃)₂•H₂O, Co(NO₃)₂•6H₂O, Al(NO₃)₃•6H₂O, and the like can be illustrated.

The hydroxamic acid group-containing molecule used in the present invention is not particularly limited as long as it is a molecule that comprises at least one or more unsubstituted or substituted hydroxamic acid groups in the molecule, further has in the molecule one or more sites capable of being bonded to the multivalent metal ion, and can be bonded to the multivalent metal ion to construct a MOF.

In the "unsubstituted or substituted hydroxamic acid group", the "unsubstituted hydroxamic acid group" is represented by the following formula (IV-1), and the "substituted hydroxamic acid group" represents any of groups of the following formulas (IV-2) to (IV-4). 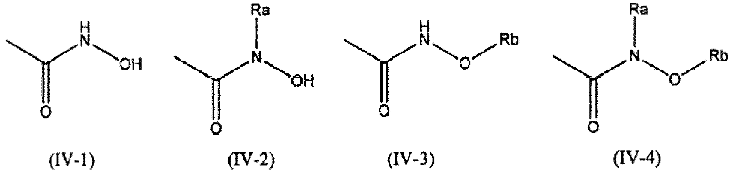 wherein Ra and Rb each independently are a functional group other than a hydrogen atom, specific examples include a C1-6 alkyl group, a C6-10 aryl group, a C1-6 alkylcarbonyl group, and a C6-10 arylcarbonyl group, and further Ra may be bonded to carbon adjacent to the carbon to which the carbonyl group is bonded, to form a ring. The C1-6 of the C1-6 alkylcarbonyl group represents the number of carbon atoms of the alkyl of the alkylcarbonyl group, and the C6-10 of the C6-10 arylcarbonyl group represents the number of carbon atoms of the aryl, and neither includes the carbon of the carbonyl group. The same applies hereinafter.

The C1-6 alkyl group may be linear or branched, and specifically, a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methyl-n-butyl group, an i-hexyl group, and the like can be illustrated.

The C6-10 aryl group may be either of a monocyclic ring and a polycyclic ring, and in the polycyclic aryl group, when at least one ring is an aromatic ring, the remaining ring may be any of a saturated alicyclic ring, an unsaturated alicyclic ring, or an aromatic ring. Specific examples include a phenyl group, a 1-naphthyl group, a 2-naphthyl group, an azulenyl group, an indenyl group, an indanyl group, and a tetralinyl group.

Examples of the C1-6 alkylcarbonyl group include an acetyl group, a n-propionyl group, an isopropionyl group, a n-butyryl group, an isobutyryl group, a pivaloyl group, and a n-pentanoyl group.

Examples of the C6-10 arylcarbonyl group include a benzoyl group, a 1-naphthylcarbonyl group, and a 2-naphthylcarbonyl group.

Examples of the structure when "Ra may be bonded to carbon adjacent to the carbon to which the carbonyl group is bonded, to form a ring" include the following structures:

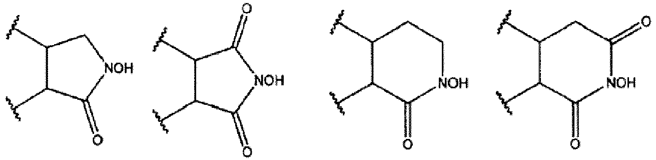

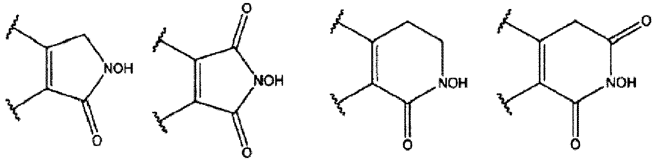

The bond in the "sites capable of being bonded to the multivalent metal ion" means a chemical bond such as an ionic bond or a coordinate bond between the metal ion and the molecule.

Specific examples of the site capable of being bonded to the multivalent metal ion preferably include the nitrogen atom in the unsubstituted or substituted hydroxamic acid group or a nitrogen-containing heterocyclic group.

Examples of the unsubstituted or substituted hydroxamic acid group include those similar to the above hydroxamic acid groups.

Specific examples of the "nitrogen-containing heterocyclic group" in "the nitrogen atom in a nitrogen-containing heterocyclic group" include a 3-pyrrolyl group, a 2-imidazolyl group, a 3-pyrazolyl group, a 2-oxazolyl group, a 2-thiazolyl group, a 3-isoxazolyl group, a 3-isothiazolyl group, a 1,2,3-triazol-4-yl group, a 1,2,4-triazol-3-yl group, a 1,2,3-oxadiazolyl-4-yl group, a 1,2,4-thiadiazolyl-4-yl group, a 1,2,4-oxadiazolyl-3-yl group, a 1,2,4-thiadiazolyl-3-yl group, a 1,3,4-oxadiazolyl-2-yl group, a 1,3,4-thiadiazolyl-2-yl group, a 4-pyridyl group, a 3-pyridyl group, a 2-pyridyl group, a 4-pyrimidyl group, a 3-pyridazyl group, a 2-pyrazyl group, a 1,3,5-triazyl-2-yl group, a 3-pyrrolidyl group, and a 4-piperidyl group.

The hydroxamic acid group-containing molecule used in the present invention is more specifically preferably at least one compound selected from a group of compounds of formulas (I) to (III). One hydroxamic acid group-containing molecule can be used singly, or two or more hydroxamic acid group-containing molecules can be mixed and used.

In formulas (I) to (III), R¹, R², R⁴, R⁶, and R⁷ each independently are a hydrogen atom, a C1-6 alkyl group, a C6-10 aryl group, a C1-6 alkylcarbonyl group, or a C6-10 arylcarbonyl group and optionally form a ring together with carbon adjacent to the carbon of the ring to which C(=O)N(R^{u})OH (u = 1, 2, 4, 6, or 7) is bonded.

As the C1-6 alkyl group, the C6-10 aryl group, the C1-6 alkylcarbonyl group, and the C6-10 arylcarbonyl group, specifically, those illustrated as the specific examples of the above Ra and similar to the specific examples can be illustrated.

Also for "optionally form a ring together with carbon adjacent to the carbon of the ring to which C(=O)N(R^{u})OH (u = 1, 2, 4, 6, or 7) is bonded", those similar to those illustrated for the above Ra can be illustrated.

R³, R⁵, R⁸, and R⁹ each independently are a C1-6 alkyl group, a C3-8 cycloalkyl group, a C6-10 aryl group, a three-to six-membered heterocyclyl group, a C1-6 alkoxy group, a C6-10 aryloxy group, a heteroaryloxy group, a halogeno group, a C1-6 haloalkyl group, a C6-10 haloaryl group, a C1-6 haloalkoxy group, a C1-6 alkylthio group, a C6-10 arylthio group, a heteroarylthio group, a C1-6 alkylsulfinyl group, a C6-10 arylsulfinyl group, a heteroarylsulfinyl group, a C1-6 alkylsulfonyl group, a C6-10 arylsulfonyl group, a heteroarylsulfonyl group, a cyano group, a nitro group, or a group represented by NR¹¹R¹² (wherein R¹¹ and R¹² each independently are a hydrogen atom, a C1-6 alkyl group, a C6-10 aryl group, a C1-6 alkylcarbonyl group, or a C6-10 arylcarbonyl group).

The C1-6 alkyl group may be linear or branched, and examples of the C1-6 alkyl group include a methyl group, an ethyl group, a n-propyl group, a n-butyl group, a n-pentyl group, a n-hexyl group, an i-propyl group, an i-butyl group, a s-butyl group, a t-butyl group, an i-pentyl group, a neopentyl group, a 2-methyl-n-butyl group, and an i-hexyl group.

Examples of the C3-8 cycloalkyl group include a cyclopropyl group, a cyclobutyl group, a cyclopentyl group, a cyclohexyl group, and a cubanyl group.

The C6-10 aryl group may be either of a monocyclic ring and a polycyclic ring, and in the polycyclic aryl group, when at least one ring is an aromatic ring, the remaining ring may be any of a saturated alicyclic ring, an unsaturated alicyclic ring, or an aromatic ring. Specific examples include a phenyl group, a 1-naphthyl group, a 2-naphthyl group, an azulenyl group, an indenyl group, an indanyl group, and a tetralinyl group.

The three- to six-membered heterocyclyl group comprises, as a constituent atom of the ring, 1 to 4 heteroatoms selected from the group consisting of a nitrogen atom, an oxygen atom, and a sulfur atom. The heterocyclyl group may be either of a monocyclic ring and a polycyclic ring. In the polycyclic heterocyclyl group, when at least one ring is a heterocycle, the remaining ring may be any of a hydrocarbon ring of a saturated alicyclic ring, an unsaturated alicyclic ring, or an aromatic ring. Examples of the three- to six-membered heterocyclyl group include a three- to six-membered saturated heterocyclyl group, a five-to six-membered heteroaryl group, and a five- to six-membered partially unsaturated heterocyclyl group.

Examples of the three- to six-membered saturated heterocyclyl group include an aziridinyl group, an epoxy group, a pyrrolidinyl group, a tetrahydrofuryl group, a thiazolidinyl group, a piperidyl group, a piperazinyl group, a morpholinyl group, a dioxolanyl group, and a dioxanyl group.

Examples of the five-membered heteroaryl group include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, a tetrazolyl group, an indonyl group, an isoindolinyl group, an indolizinyl group, a benzimidazolyl group, and a carbazolyl group.

Examples of the six-membered heteroaryl group can include a pyridyl group, a pyrazyl group, a pyrimidyl group, a pyridazyl group, a triazyl group, a quinolinyl group, an isoquinolinyl group, a quinoxalinyl group, a cinnolinyl group, a quinazolyl group, a phthalazinyl group, an acridinyl group, a naphthazinyl group, and a phenazinyl group.

Examples of the C1-6 alkoxy group include a methoxy group, an ethoxy group, a n-propoxy group, an i-propoxy group, a n-butoxy group, a s-butoxy group, an i-butoxy group, and a t-butoxy group.

Examples of the C6-10 aryloxy group include a phenoxy group, a 1-naphthyloxy group, a 2-naphthyloxy group, an azulenyloxy group, an indenyloxy group, an indanyloxy group, and a tetralinyloxy group.

Examples of the heteroaryloxy group include a furyloxy group, a thiazolyloxy group, and a pyridyloxy group.

Examples of the halogeno group include a fluoro group, a chloro group, a bromo group, and an iodo group.

Examples of the C1-6 haloalkyl group include a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group, and a 2,4,6-trichlorohexyl group.

Examples of the C6-10 haloaryl group include a 4-chlorophenyl group, a 3,5-dichlorophenyl group, a 2,4,6-trichlorophenyl group, and a 2,3,4,5,6-pentafluorophenyl group.

Examples of the C1-6 haloalkoxy group include a trifluoromethoxy group, a 2,2,2-trifluoroethoxy group, a pentafluoroethoxy group, a 3,3,3-trifluoropropoxy group, a 2,2,3,3,3-pentafluoropropoxy group, a perfluoropropoxy group, a 2,2,2-trifluoro-1-trifluoromethylethoxy group, a perfluoroisopropoxy group, a 4-fluorobutoxy group, a 2,2,3,3,4,4,4-heptafluorobutoxy group, a perfluorobutoxy group, a perfluoropentoxy group, a perfluorohexyloxy group, a 2,2,2-trichloroethoxy group, a 4-chlorobutoxy group, a perchlorohexyloxy group, and a 2,4,6-trichlorohexyloxy group.

Examples of a C1-6 alkylsulfanyl group include a methylsulfanyl group, an ethylsulfanyl group, a n-propylsulfanyl group, an i-propylsulfanyl group, a n-butylsulfanyl group, an i-butylsulfanyl group, a s-butylsulfanyl group, and a t-butylsulfanyl group.

Examples of a C6-10 arylsulfanyl group include a phenylsulfanyl group, a 1-naphthylsulfanyl group, a 2-naphthylsulfanyl group, an azulenylsulfanyl group, an indenylsulfanyl group, an indanylsulfanyl group, and a tetralinylsulfanyl group.

Examples of a heteroarylsulfanyl group include a furylsulfanyl group, a thiazolylsulfanyl group, and a pyridylsulfanyl group.

Examples of the C1-6 alkylsulfinyl group include a methylsulfinyl group, an ethylsulfinyl group, and a t-butylsulfinyl group.

Examples of the C6-10 arylsulfinyl group include a phenylsulfinyl group, a 1-naphthylsulfinyl group, a 2-naphthylsulfinyl group, an azulenylsulfinyl group, an indenylsulfinyl group, an indanylsulfinyl group, and a tetralinylsulfinyl group.

Examples of the heteroarylsulfinyl group include a furylsulfinyl group, a thiazolylsulfinyl group, and a pyridylsulfinyl group.

Examples of the C1-6 alkylsulfonyl group include a methylsulfonyl group, an ethylsulfonyl group, and a t-butylsulfonyl group.

Examples of the C6-10 arylsulfonyl group include a phenylsulfonyl group, a 1-naphthylsulfonyl group, a 2-naphthylsulfonyl group, an azulenylsulfonyl group, an indenylsulfonyl group, an indanylsulfonyl group, and a tetralinylsulfonyl group.

Examples of the heteroarylsulfonyl group include a furylsulfonyl group, a thiazolylsulfonyl group, and a pyridylsulfonyl group.

In the group represented by NR¹¹R¹², R¹¹ and R¹² each independently are a hydrogen atom, a C1-6 alkyl group, a C6-10 aryl group, a C1-6 alkylcarbonyl group, or a C6-10 arylcarbonyl group.

Examples of the C1-6 alkyl group and the C6-10 aryl group include those similar to those illustrated for the above R³, R⁵, R⁸, and R⁹.

Examples of the C1-6 alkylcarbonyl group and the C6-10 arylcarbonyl group include those similar to those illustrated for the above Ra.

Examples of the group represented by NR¹¹R¹² include an amino group, a methylamino group, a dimethylamino group, an ethyl-i-propylamino group, an anilino group, a diphenylamino group, an acetylamino group, and a benzoylamino group.

In formula (II), A is a five- or six-membered aromatic heterocycle containing 1 to 4 nitrogen atoms in an integer number as a ring-constituting atom and may be either of a monocyclic ring and a polycyclic ring. However, in the case of a polycyclic ring, at least one ring is a heterocycle, and the remaining ring is any of a hydrocarbon ring of a saturated alicyclic ring, an unsaturated alicyclic ring, or an aromatic ring. Examples of such an aromatic heterocycle include a pyrrolyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, a tetrazolyl group, a pyridyl group, a pyrazyl group, a pyrimidyl group, a pyridazyl group, a triazyl group, an indonyl group, an isoindolinyl group, an indolizinyl group, a benzimidazolyl group, a carbazolyl group, a quinolinyl group, an isoquinolinyl group, a quinoxalinyl group, a cinnolinyl group, a quinazolyl group, a phthalazinyl group, an acridinyl group, a naphthazinyl group, and a phenazinyl group.

The C1-6 alkyl group, the C6-10 aryl group, the C1-6 alkylcarbonyl group, or the C6-10 arylcarbonyl group for R¹, R², R⁴, R⁶, and R⁷; and the C1-6 alkyl group, the C3-8 cycloalkyl group, the C6-10 aryl group, the three- to six-membered heterocyclyl group, the C1-6 alkoxy group, the C6-10 aryloxy group, the heteroaryloxy group, the halogeno group, the C1-6 haloalkyl group, the C6-10 haloaryl group, the C1-6 haloalkoxy group, the C1-6 alkylthio group, the C6-10 arylthio group, the heteroarylthio group, the C1-6 alkylsulfinyl group, the C6-10 arylsulfinyl group, the heteroarylsulfinyl group, the C1-6 alkylsulfonyl group, the C6-10 arylsulfonyl group, the heteroarylsulfonyl group, or the group represented by NR¹¹R¹² (wherein R¹¹ and R¹² each independently are a hydrogen atom, a C1-6 alkyl group, a C6-10 aryl group, a C1-6 alkylcarbonyl group, or a C6-10 arylcarbonyl group) for R³, R⁵, R⁸, and R⁹ can further have a substituent in a chemically allowed range as needed.

As such a substituent, the groups shown below can be illustrated.

A C1-6 alkyl group such as a methyl group, an ethyl group, a n-propyl group, an i-propyl group, a n-butyl group, a s-butyl group, an i-butyl group, a t-butyl group, a n-pentyl group, or a n-hexyl group;
a C2-6 alkenyl group such as a vinyl group, a 1-propenyl group, a 2-propenyl group (allyl group), a propen-2-yl group, a 1-butenyl group, a 2-butenyl group, a 3-butenyl group, a 1-methyl-2-propenyl group, or a 2-methyl-2-propenyl group;
a C2-6 alkynyl group such as an ethynyl group, a 1-propynyl group, a 2-propynyl group (propargyl group), a 1-butynyl group, a 2-butynyl group, a 3-butynyl group, or a 1-methyl-2-propynyl group;
a C3-8 cycloalkyl group such as a cyclopropyl group, a cyclobutyl group, a cyclopentyl group, a cyclohexyl group, or a cubanyl group;
a C6-10 aryl group such as a phenyl group or a naphthyl group;
a C6-10 aryl C1-6 alkyl group such as a benzyl group or a phenethyl group;
a three- to six-membered heterocyclyl group;
a three- to six-membered heterocyclyl C1-6 alkyl group;
an oxo group;
a hydroxyl group;
a C1-6 alkoxy group such as a methoxy group, an ethoxy group, a n-propoxy group, an i-propoxy group, a n-butoxy group, a s-butoxy group, an i-butoxy group, or a t-butoxy group;
a C2-6 alkenyloxy group such as a vinyloxy group, an allyloxy group, a 1-propenyloxy group, a propen-2-yloxy group, a 3-butenyloxy group, or a 2-butenyloxy group;
a C2-6 alkynyloxy group such as an ethynyloxy group or a propargyloxy group;
a C6-10 aryloxy group such as a phenoxy group or a naphthoxy group;
a C6-10 aryl C1-6 alkoxy group such as a benzyloxy group or a phenethyloxy group;
a five- to six-membered heteroaryloxy group such as a thiazolyloxy group or a pyridyloxy group;
a five- to six-membered heteroaryl C1-6 alkyloxy group such as a thiazolylmethyloxy group or a pyridylmethyloxy group;
a formyl group;
a C1-6 alkylcarbonyl group such as an acetyl group or a propionyl group;
a formyloxy group;
a C1-6 alkylcarbonyloxy group such as an acetyloxy group or a propionyloxy group;
a C6-10 arylcarbonyl group such as a benzoyl group;
a C1-6 alkoxycarbonyl group such as a methoxycarbonyl group, an ethoxycarbonyl group, a n-propoxycarbonyl group, an i-propoxycarbonyl group, a n-butoxycarbonyl group, or a t-butoxycarbonyl group;
a C1-6 alkoxycarbonyloxy group such as a methoxycarbonyloxy group, an ethoxycarbonyloxy group, a n-propoxycarbonyloxy group, an i-propoxycarbonyloxy group, a n-butoxycarbonyloxy group, or a t-butoxycarbonyloxy group;
a carboxy group;
a halogeno group such as a fluoro group, a chloro group, a bromo group, or an iodo group;
a C1-6 haloalkyl group such as a fluoromethyl group, a difluoromethyl group, a trifluoromethyl group, a 2,2,2-trifluoroethyl group, a pentafluoroethyl group, a 3,3,3-trifluoropropyl group, a 2,2,3,3,3-pentafluoropropyl group, a perfluoropropyl group, a 2,2,2-trifluoro-1-trifluoromethylethyl group, a perfluoroisopropyl group, a 4-fluorobutyl group, a 2,2,3,3,4,4,4-heptafluorobutyl group, a perfluorobutyl group, a perfluoropentyl group, a perfluorohexyl group, a chloromethyl group, a bromomethyl group, a dichloromethyl group, a dibromomethyl group, a trichloromethyl group, a tribromomethyl group, a 1-chloroethyl group, a 2,2,2-trichloroethyl group, a 4-chlorobutyl group, a perchlorohexyl group, or a 2,4,6-trichlorohexyl group;
a C2-6 haloalkenyl group such as a 2-chloro-1-propenyl group or a 2-fluoro-1-butenyl group;
a C2-6 haloalkynyl group such as a 4,4-dichloro-1-butynyl group, a 4-fluoro-1-pentynyl group, or a 5-bromo-2-pentynyl group;
a C1-6 haloalkoxy group such as a trifluoromethoxy group, a 2-chloro-n-propoxy group, or a 2,3-dichlorobutoxy group;
a C2-6 haloalkenyloxy group such as a 2-chloropropenyloxy group or a 3-bromobutenyloxy group;
a C1-6 haloalkylcarbonyl group such as a chloroacetyl group, a trifluoroacetyl group, or a trichloroacetyl group;
an amino group;
a C1-6 alkyl-substituted amino group such as a methylamino group, a dimethylamino group, or a diethylamino group;
a C6-10 arylamino group such as an anilino group or a naphthylamino group;
a C6-10 aryl C1-6 alkylamino group such as a benzylamino group or a phenethylamino group;
a formylamino group;
a C1-6 alkylcarbonylamino group such as an acetylamino group, a propanoylamino group, a butyrylamino group, or an i-propylcarbonylamino group;
a C1-6 alkoxycarbonylamino group such as a methoxycarbonylamino group, an ethoxycarbonylamino group, a n-propoxycarbonylamino group, or an i-propoxycarbonylamino group;
   a C1-6 alkylsulfoxyimino group such as a S,S-dimethylsulfoxyimino group;
an aminocarbonyl group unsubstituted or having a substituent, such as an aminocarbonyl group, a dimethylaminocarbonyl group, a phenylaminocarbonyl group, or a N-phenyl-N-methylaminocarbonyl group;
an imino C1-6 alkyl group such as an iminomethyl group, a 1-iminoethyl group, or a 1-imino-n-propyl group;
a substituted or unsubstituted N-hydroxyimino C1-6 alkyl group such as a N-hydroxy-iminomethyl group, a 1-(N-hydroxyimino)ethyl group, a 1-(N-hydroxyimino)propyl group, a N-methoxyiminomethyl group, or a 1-(N-methoxyimino)ethyl group;
a hydroxyimino group;
a C1-6 alkoxyimino group such as a methoxyimino group, an ethoxyimino group, a n-propoxyimino group, an i-propoxyimino group, or a n-butoxyimino group;
an aminocarbonyloxy group;
a C1-6 alkyl-substituted aminocarbonyloxy group such as an ethylaminocarbonyloxy group or a dimethylaminocarbonyloxy group;
a thioxo group;
a sulfanyl group;
a C1-6 alkylsulfanyl group such as a methylsulfanyl group, an ethylsulfanyl group, a n-propylsulfanyl group, an i-propylsulfanyl group, a n-butylsulfanyl group, an i-butylsulfanyl group, a s-butylsulfanyl group, or a t-butylsulfanyl group;
a C1-6 haloalkylsulfanyl group such as a trifluoromethylsulfanyl group or a 2,2,2-trifluoroethylsulfanyl group;
a C6-10 arylsulfanyl group such as a phenylsulfanyl group or a naphthylsulfanyl group;
a five- to six-membered heteroarylsulfanyl group such as a thiazolylsulfanyl group or a pyridylsulfanyl group;
a C1-6 alkylsulfinyl group such as a methylsulfinyl group, an ethylsulfinyl group, or a t-butylsulfinyl group;
a C1-6 haloalkylsulfinyl group such as a trifluoromethylsulfinyl group or a 2,2,2-trifluoroethylsulfinyl group;
a C6-10 arylsulfinyl group such as a phenylsulfinyl group;
a five- to six-membered heteroarylsulfinyl group such as a thiazolylsulfinyl group or a pyridylsulfinyl group;
a C1-6 alkylsulfonyl group such as a methylsulfonyl group, an ethylsulfonyl group, or a t-butylsulfonyl group;
a C1-6 haloalkylsulfonyl group such as a trifluoromethylsulfonyl group or a 2,2,2-trifluoroethylsulfonyl group;
a C6-10 arylsulfonyl group such as a phenylsulfonyl group;
a five- to six-membered heteroarylsulfonyl group such as a thiazolylsulfonyl group or a pyridylsulfonyl group;
a sulfo group;
a C1-6 alkylsulfonyloxy group such as a methylsulfonyloxy group, an ethylsulfonyloxy group, or a t-butylsulfonyloxy group;
a C1-6 haloalkylsulfonyloxy group such as a trifluoromethylsulfonyloxy group or a 2,2,2-trifluoroethylsulfonyloxy group;
a tri-C1-6 alkyl-substituted silyl group such as a trimethylsilyl group, a triethylsilyl group, or a t-butyldimethylsilyl group;
a tri-C6-10 aryl-substituted silyl group such as a triphenylsilyl group;
a C2-C6 alkenyl C1-C6 dialkyl-substituted silyl group such as an allyldimethylsilyl group or a vinyldimethylsilyl group;
a C1-C6 alkyl di-C6-C10 aryl-substituted silyl group such as a t-butyldiphenylsilyl group or a diphenylmethylsilyl group;
a di-C1-C6 alkyl C6-C10 aryl-substituted silyl group such as a dimethylphenylsilyl group;
a (C6-C10 phenyl C1-C6 alkyl)di-C1-C6 alkylsilyl group such as a benzyldimethylsilyl group or a 3-phenylpropyldimethylsilyl group;
a C1-C6 alkyl C6-C10 phenyl C2-C6 alkenylsilyl group such as a methylphenylvinylsilyl group;
a tri-C1-C6 alkoxy-substituted silyl group such as a trimethoxysilyl group or a triethoxysilyl group;
a di-C1-C6 alkyl-substituted silyl group such as a dimethylsilyl group or a diethylsilyl group;
a di-C1-C6 alkoxy-substituted silyl group such as a dimethoxysilyl group or a diethoxysilyl group;
a C1-C6 alkoxy C1-C6 alkyl-substituted silyl group such as a methoxydimethylsilyl group;
a C1-C6 alkoxy C6-C10 aryl-substituted silyl group such as a t-butoxydiphenylsilyl group;
a C1-C6 alkyl di-C1-C6 alkoxy-substituted silyl group such as a methyldimethoxysilyl group;
a cyano group; and
a nitro group.

The above "three- to six-membered heterocyclyl group" comprises, as a constituent atom of the ring, 1 to 4 heteroatoms selected from the group consisting of a nitrogen atom, an oxygen atom, and a sulfur atom. The heterocyclyl group may be either of a monocyclic ring and a polycyclic ring. In the polycyclic heterocyclyl group, when at least one ring is a heterocycle, the remaining ring may be any of a hydrocarbon ring of a saturated alicyclic ring, an unsaturated alicyclic ring, or an aromatic ring. Examples of the "three- to six-membered heterocyclyl group" can include a three- to six-membered saturated heterocyclyl group, a five- to six-membered heteroaryl group, and a five-to six-membered partially unsaturated heterocyclyl group.

Examples of the three- to six-membered saturated heterocyclyl group can include an aziridinyl group, an epoxy group, a pyrrolidinyl group, a tetrahydrofuryl group, a thiazolidinyl group, a piperidyl group, a piperazinyl group, a morpholinyl group, a dioxolanyl group, and a dioxanyl group.

Examples of the five-membered heteroaryl group can include a pyrrolyl group, a furyl group, a thienyl group, an imidazolyl group, a pyrazolyl group, an oxazolyl group, an isoxazolyl group, a thiazolyl group, an isothiazolyl group, a triazolyl group, an oxadiazolyl group, a thiadiazolyl group, a tetrazolyl group, an indonyl group, an isoindolinyl group, an indolizinyl group, a benzimidazolyl group, and a carbazolyl group.

Examples of the six-membered heteroaryl group can include a pyridyl group, a pyrazinyl group, a pyrimidinyl group, a pyridazinyl group, a triazinyl group, a quinolinyl group, an isoquinolinyl group, a quinoxalinyl group, a cinnolinyl group, a quinazolyl group, a phthalazinyl group, an acridinyl group, a naphthazinyl group, and a phenazinyl group.

Examples of the five- to six-membered partially unsaturated heterocyclyl group can include an isoxazolinyl group and a pyrazolinyl group.

Examples of the three- to six-membered heterocyclyl 1-6 alkyl group can include a glycidyl group, a 2-tetrahydrofurylmethyl group, a 2-pyrrolylmethyl group, a 2-imidazolylmethyl group, a 3-isoxazolylmethyl group, a 5-isoxazolylmethyl group, a 2-pyridylmethyl group, a 4-pyridylmethyl group, and a 3-isoxazolinylmethyl group.

Specific examples of the compound of formula (I) include compounds of the following formulas:

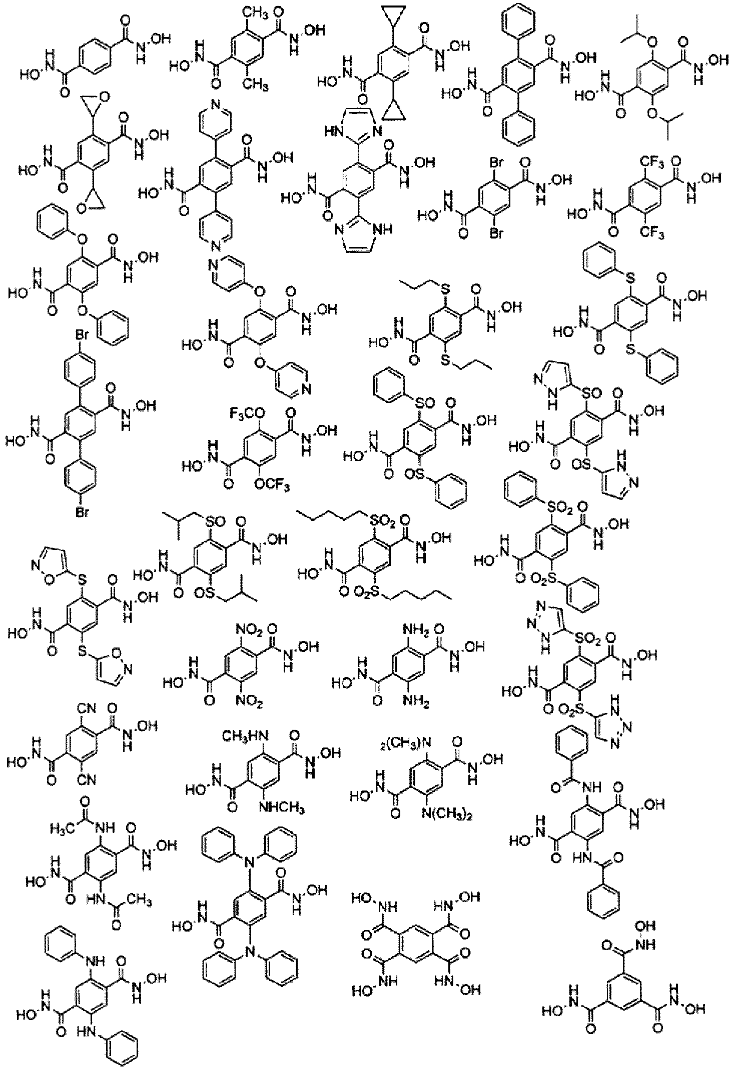

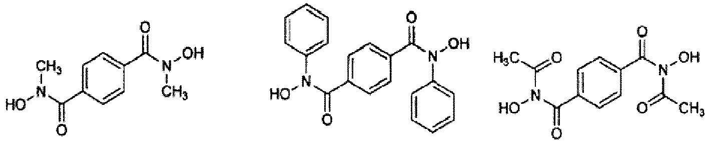

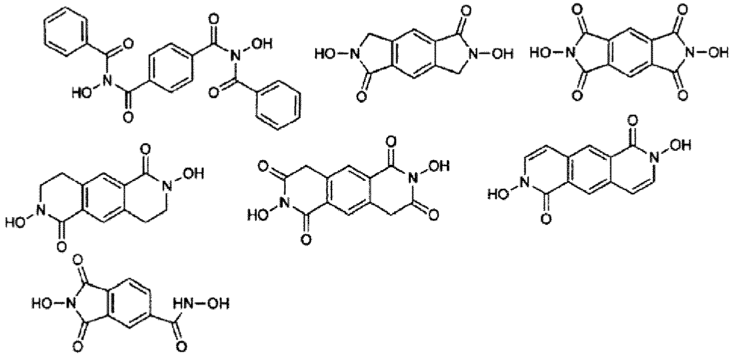

Specific examples of the compound of formula (II) include compounds of the following formulas:

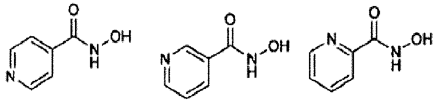

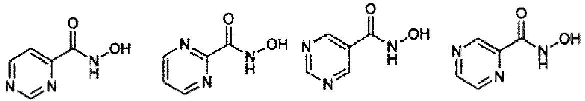

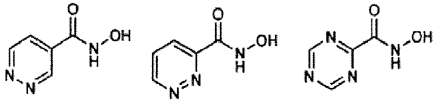

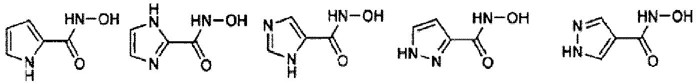

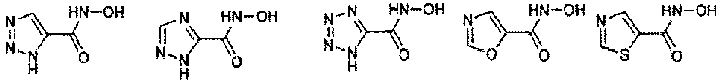

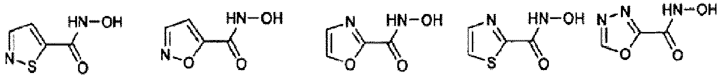

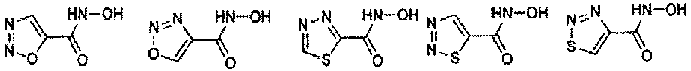

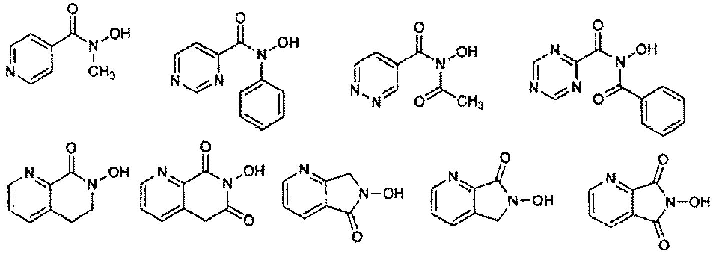

Specific examples of the compound of formula (III) include compounds of the following formulas:

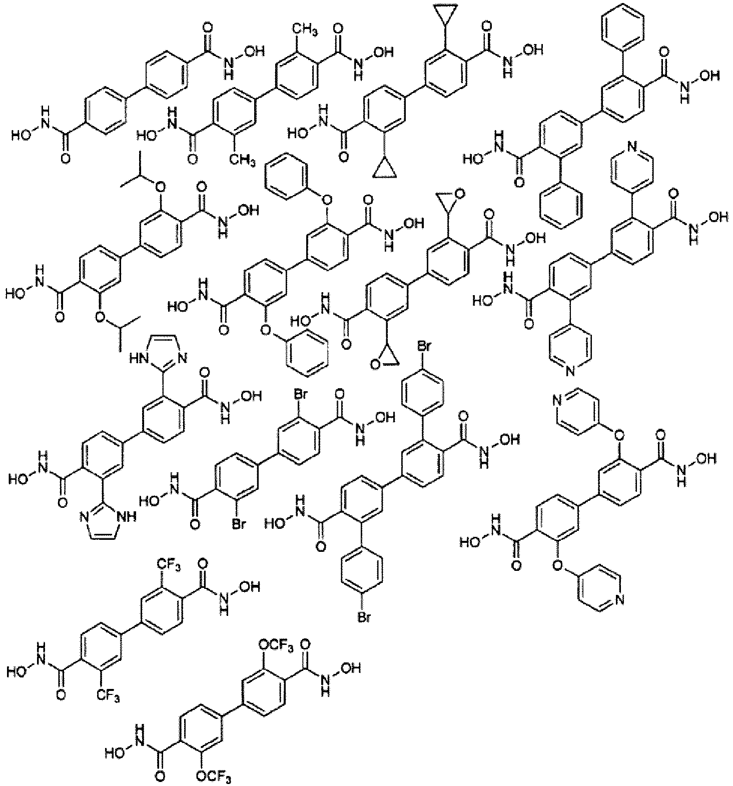

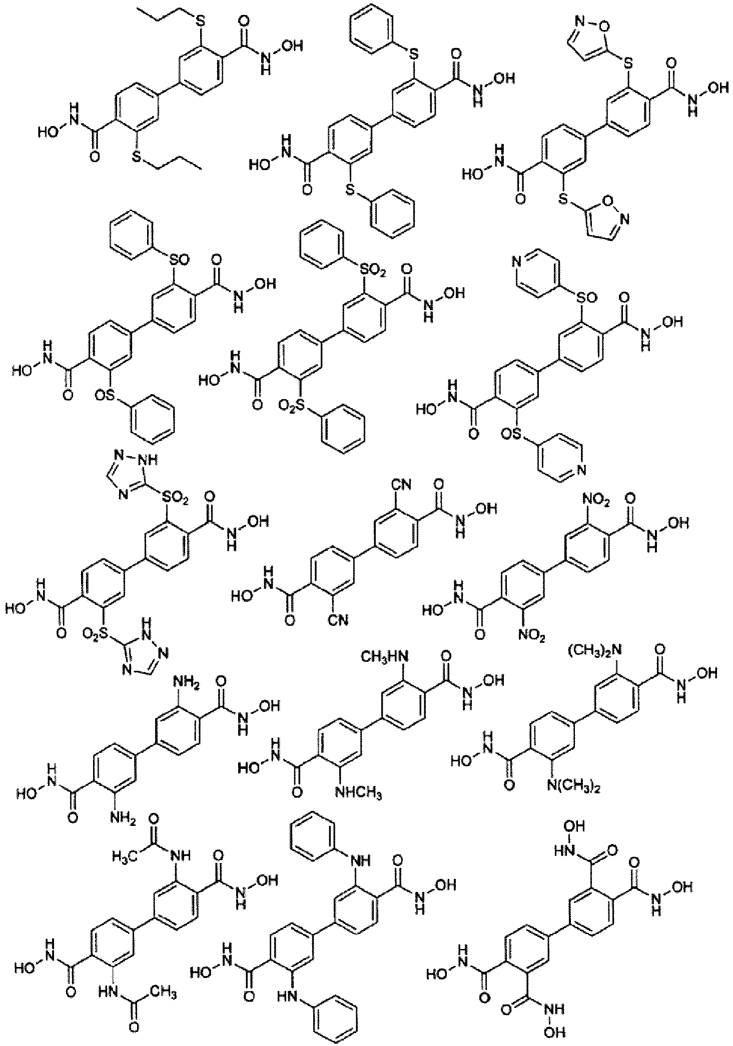

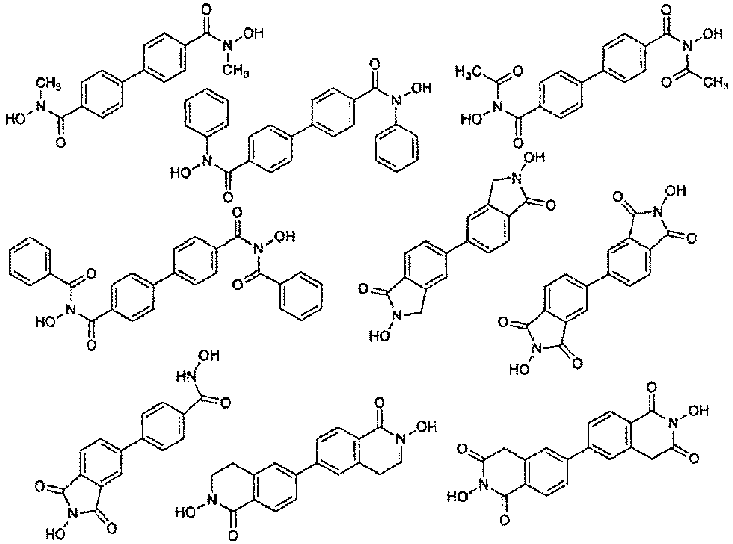

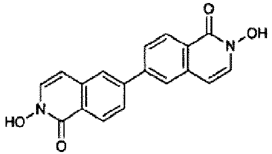

The storage material for a gas other than nitrogen according to the present invention comprises a metal-organic framework comprising a multivalent metal ion and a hydroxamic acid group-containing molecule bonded to each other. The bond in the "metal-organic framework comprising a multivalent metal ion and a hydroxamic acid group-containing molecule bonded to each other" means a chemical bond such as an ionic bond or a coordinate bond between the multivalent metal ion and the hydroxamic acid group-containing molecule.

In the metal-organic framework used for the storage material for a gas other than nitrogen according to the present invention, in addition to the hydroxamic acid group-containing molecule, a molecule comprising a nitrogen atom (excluding the hydroxamic acid group-containing molecule) (hereinafter referred to as a nitrogen atom-containing molecule) can be contained as a constituent unit. Examples of such a molecule can include isonicotinic acid, benzimidazole, imidazole, 1,4-diazabicyclo[2.2.2]octane (DABCO), pyrazine, 4,4'-dipyridyl, 1,2-di(4-pyridyl)ethylene, 2,7-diazapyrene, 4,4'-azobispyridine, and bis(3-(4-pyridyl)-2,4-pentanedionato)copper.

The mixing molar ratio when the hydroxamic acid group-containing molecule and the nitrogen atom-containing molecule are used is not particularly limited, but, for example, when the nitrogen atom-containing molecule is used as a pillar molecule, and crosslinking is performed with the pillar molecule to construct a three-dimensional structure like a pillared layer type, the nitrogen atom-containing molecule is preferably used in excess of the hydroxamic acid group-containing molecule.

The method for producing the metal-organic framework used for the storage material for a gas other than nitrogen according to the present invention is not particularly limited, and it is possible to use any of a solution method such as a solvent diffusion method, a solvent stirring method, or a hydrothermal method, a microwave method in which a reaction solution is irradiated with microwaves to uniformly heat the entire system in a short time, an ultrasonic method in which by irradiating a reaction container with ultrasonic waves, changes in pressure occur repeatedly in the reaction container, and due to these changes in pressure, a phenomenon called cavitation in which a solvent forms bubbles and collapses occurs, and at the time, energy fields as high as about 5000 K and 10000 bar are reaction fields for the production of crystals locally formed, a solid phase synthesis method in which a metal ion source and an organic ligand are mixed without using a solvent, a LAG (liquid assisted grinding) method in which a metal ion source and a hydroxamic acid group-containing molecule are mixed with water at the water-of-crystallization level added, and the like.

The production method includes, for example, the step of preparing each of a first solution containing a metal compound that is a source of a metal ion, and a solvent, a second solution containing a hydroxamic acid group-containing molecule and a solvent, and a third solution containing a compound that is another polydentate ligand, and a solvent, as needed, and the step of mixing the first solution with the second solution and the third solution to prepare a reaction liquid, and heating this reaction liquid to obtain a metal-organic framework. The first to third solutions need not be separately prepared, and, for example, the above metal compound, the hydroxamic acid group-containing molecule, the compound that is another polydentate ligand, and the solvent may be mixed at one time to prepare one solution.

The mixing molar ratio between the above metal compound and the hydroxamic acid group-containing molecule can be arbitrarily selected in accordance with the pore size and surface characteristics of the metal-organic framework to be obtained, but 1 mol or more of the metal compound is preferably used based on 1 mol of the hydroxamic acid group-containing molecule, and further 1.1 mol or more, further 1.2 mol or more, further 1.5 mol or more, further 2 mol or more, and further 3 mol or more of the metal compound is preferably used.

The concentration of the above metal ion in the reaction liquid is preferably in the range of 25 to 200 mol/L.

The concentration of the hydroxamic acid group-containing molecule in the reaction liquid is preferably in the range of 10 to 100 mol/L.

The concentration of the organic ligand other than the hydroxamic acid group-containing molecule in the reaction liquid is preferably 25 to 100 mol/L.

As the solvent used, one or more solvents selected from the group consisting of N,N-dimethylformamide (hereinafter sometimes described as "DMF"), N,N-diethylformamide (hereinafter sometimes described as "DEF"), N,N-dimethylacetamide (hereinafter sometimes described as "DMA"), and water can be used. It is preferable that among these, any of N,N-dimethylformamide, N,N-diethylformamide, or N,N-dimethylacetamide be used singly, or a N,N-dimethylformamide/water mixed solvent, a N,N-diethylformamide/water mixed solvent, or a N,N-dimethylacetamide/water mixed solvent be used.

The heating temperature of the reaction liquid is not particularly limited but is preferably in the range of room temperature to 140°C.

The gas to be stored in the storage material for a gas other than nitrogen according to the present invention is not particularly limited as long as it is a gas that can be stored in the material. Specific examples include hydrogen, carbon dioxide, methane, and acetylene, and among them, hydrogen and carbon dioxide are preferable. However, nitrogen is excluded. A "gas" hereinafter refers to a gas other than nitrogen.

The method for storing a gas using the gas storage material of the present invention is not particularly limited, but a method of bringing the gas storage material of the present invention and a gas into contact with each other is preferable, and the contact method is not particularly limited. Examples of the method include a method of filling a tank with the gas storage material of the present invention to provide a gas storage tank, and allowing a gas to flow into the tank, a method of supporting the gas storage material of the present invention on the surface constituting the inner wall of a tank to provide a gas storage tank, and allowing a gas to flow into the tank, and a method of molding a tank from a material comprising the gas storage material of the present invention to provide a gas storage tank, and allowing a gas to flow into the tank.

The gas storage tank of the present invention is hermetically molded from a raw material that can withstand normal pressure or high pressure, so as to have a space that can store a gas inside, and can be constituted by filling the molded tank with the gas storage material of the present invention.

As another aspect of the gas storage tank of the present invention, the gas storage tank of the present invention can be constituted by hermetically molding a material comprising the gas storage material of the present invention in the form of having a space inside which a gas can be allowed to flow. The material comprising the gas storage material of the present invention indicates the gas storage material of the present invention itself or a material in which the gas storage material of the present invention is combined with another moldable material. These aspects may constitute the gas storage tank of the present invention singly or together.

### Examples

The present invention will be explained more specifically below referring to Examples, but the present invention is not intended to be limited to the scope of the Examples.

As hydroxamic acid group-containing molecules constituting metal-organic frameworks used for gas storage materials of the present invention, the compounds shown in the following Table 1 were used.

**[Table 1]**

| | | | |
|---|---|---|---|
| Compound number | **1** | **2** | **3** |
| Structural formula | 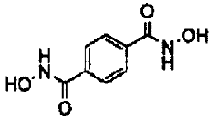 | 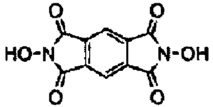 | 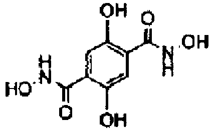 |
| Compound number | **4** | **5** | **6** |
| Structural formula | 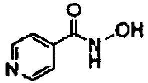 | 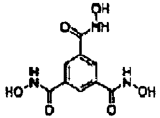 | 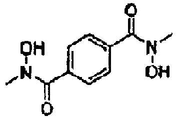 |
| Compound number | **7** | **8** | **9** |
| Structural formula | 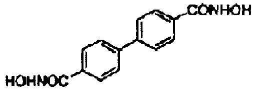 | 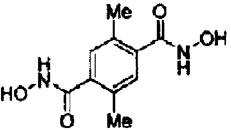 | 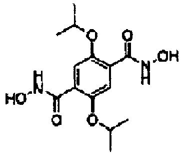 |
| Compound number | **10** | | |
| Structural formula | 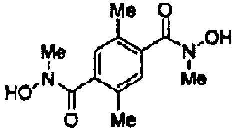 | | |

### (Reference Example 1) Synthesis of Compound of Compound Number 9

Diethyl 2,5-dihydroxyterephthalate (6.5 mmol), isopropyl bromide (25.8 mmol), potassium carbonate (138 mmol), and 20 mL of DMF were heated under nitrogen at 60°C for 24 hours. The mixture was returned to room temperature, water was added, the mixture was separated, and the organic layer extracted with chloroform was dried with magnesium sulfate and filtered. The filtrate was distilled off under reduced pressure, and the obtained solid was purified using silica gel column chromatography (chloroform). 1.6 g (4.7 mmol) of diethyl 2,5-diisopropoxyterephthalate was obtained as a colorless solid. 15 mL of a potassium hydroxide (80 mmol) methanol solution was added to a solution of hydroxylamine hydrochloride (80 mmol) in 10 mL of methanol at 0°C, and the mixture was stirred for 5 minutes. Its filtrate was added to the diethyl 2,5-diisopropoxyterephthalate, and the mixture was stirred at room temperature for 5 hours. 1 N hydrochloric acid was added until a precipitate appeared, and the deposited solid was filtered. The solid was rinsed well with ethyl acetate and water and vacuum-dried to obtain 1.4 g (4.0 mmol) of 2,5-diisopropoxy-1,4-benzene-dicarbohydroxamic acid (compound No 9).

### (Reference Example 2) Synthesis of Compound of Compound Number 10

2,5-Dimethylterephthalic acid (4.0 mmol), oxalyl chloride (10 mmol), and 10 mL of THF were stirred under nitrogen at room temperature all night. The volatile substances were distilled off under reduced pressure, and acetonitrile was added to the residue to prepare an acid chloride solution. The above-described acid chloride solution was added to a solution obtained by adding 20 mL of acetonitrile to N-methylhydroxylamine hydrochloride (8.8 mmol) and imidazole (16 mmol) and stirring the mixture for 10 minutes. The mixture was stirred at room temperature for 15 hours. The deposited solid was filtered, rinsed well with water, and vacuum-dried to obtain 0.6 g (2.4 mmol) of 2,5-dimethyl-1,4-benzene-di(N-methylcarbohydroxamic acid) (compound No 10).

### [Example 1-1]

10 mL of DMF was added to the compound of compound number 1 (0.5 mmol) and zinc nitrate hexahydrate (1.0 mmol), and the mixture was heated in an oven (reaction conditions: 120°C, 24 hours). The mixture was returned to room temperature and centrifuged, and then the supernatant was removed. After washing using 10 ml of DMF, the solvent was removed, and the solvent was exchanged for chloroform. 10 ml of chloroform was added followed by all-night immersion. The chloroform was removed, and then vacuum drying was performed at 150°C for 5 hours to obtain a metal-organic framework 1 as a light brown solid.

### [Example 1-2] to [Example 1-31]

Metal-organic frameworks 1-2 to 1-31 were obtained by performing operations similar to those of Example 1-1 except that the organic ligands and the solvents shown in the following Table 2 were used, and the reaction was performed under the reaction conditions (temperature and heating time) shown in Table 2. The results are shown in Table 2.

**[Table 2]**

| Example | Compound number | Solvent | Temperature (°C) | Heating time (hr) | Properties |
|---|---|---|---|---|---|
| **1-1** | **1** | **DMF** | **120** | **24** | Light brown solid |
| **1-2** | **1** | **DEF** | **120** | **24** | Light brown solid |
| **1-3** | **1** | **DMA** | **90** | **24** | White powder |
| **1-4** | **1** | **DEF** | **90** | **24** | White powder |
| **1-5** | **2** | **DMF** | **120** | **19** | Light yellow powder |
| **1-6** | **2** | **DEF** | **120** | **19** | Light yellow powder |
| **1-7** | **2** | **DMF** | **90** | **24** | Light yellow powder |
| **1-8** | **3** | **DMF** | **120** | **24** | Dark brown powder |
| **1-9** | **3** | **DEF** | **120** | **24** | Dark brown powder |
| **1-10** | **4** | **DMF** | **120** | **24** | White powder |
| **1-11** | **4** | **DEF** | **120** | **168** | Light peach powder |
| **1-12** | **4** | **DEF** | **90** | **332** | Off-white powder and light yellow crystals |
| **1-13** | **5** | **DEF** | **85** | **24** | Light red powder |
| **1-14** | **6** | **DMF** | **90** | **24** | White powder |
| **1-15** | **6** | **DMF** | **120** | **24** | White powder |
| **1-16** | **6** | **DEF** | **90** | **24** | White powder |
| **1-17** | **6** | **DEF** | **120** | **24** | Light yellow powder |
| **1-18** | **7** | **DMF** | **90** | **24** | Light brown powder |
| **1-19** | **7** | **DEF** | **90** | **24** | Light brown powder |
| **1-20** | **8** | **DMF** | **90** | **24** | Dark brown powder |
| **1-21** | **8** | **DMF** | **120** | **24** | Dark brown powder |
| **1-22** | **8** | **DEF** | **90** | **24** | Dark brown powder |
| **1-23** | **8** | **DEF** | **120** | **24** | Dark brown powder |
| **1-24** | **9** | **DMF** | **90** | **2 4** | Gray powder |
| **1-25** | **9** | **DMF** | **120** | **24** | Gray powder |
| **1-26** | **9** | **DEF** | **90** | **24** | Gray powder |
| **1-27** | **9** | **DEF** | **120** | **24** | Brown powder |
| **1-28** | **10** | **DMF** | **90** | **24** | White powder |
| **1-29** | 10 | **DMF** | **120** | **24** | White powder |
| **1-30** | 10 | **DEF** | **90** | **24** | White powder |
| **1-31** | **10** | **DEF** | **120** | **24** | White powder |

### [Example 2-1]

40 mL of DMF was added to the compound of compound number 1 (1.2 mmol) and zinc nitrate hexahydrate (1.6 mmol). Triethylamine (14.4 mmol) was dropped thereinto, and the mixture was stirred at room temperature for 30 minutes. The precipitate was filtered, and the precipitate that was a filtered material was washed three times with 10 mL of DMF. Subsequently, the precipitate that was a filtered material was washed three times using 10 mL of chloroform. The precipitate was immersed in 10 mL of chloroform all night, and after filtration, the solid was vacuum-dried at 150°C for 5 hours to obtain a metal-organic framework 2-1 as a white solid.

### [Example 2-2] to [Example 2-6]

Metal-organic frameworks 2-2 to 2-6 were obtained by performing operations similar to those of Example 2-1 except that the compounds and the solvents shown in the following Table 3 were used, and the reaction time shown in Table 3 was set. The results are shown in Table 3.

**[Table 3]**

| Example | Compound number | Solvent | Reaction time (hr) | Properties |
|---|---|---|---|---|
| **2-1** | **1** | **DMF** | **0. 5** | White powder |
| **2-2** | **1** | **DEF** | **0. 5** | White powder |
| **2-3** | **2** | **DMF** | **0. 5** | Brown powder |
| **2-4** | **2** | **DEF** | **0. 08** | Dark brown powder |
| **2-5** | **3** | **DMF** | **0. 5** | Dark brown powder |
| **2-6** | **4** | **DMF** | **0. 5** | Light yellow powder |

### [Example 3-1]

3 mL of DMF and 1 mL of water were added to the compound of compound number 1 (0.5 mmol), zinc nitrate hexahydrate (0.5 mmol), and isonicotinic acid (0.5 mmol), and the mixture was heated in an oven (reaction conditions: 120°C, 12 hours). The mixture was returned to room temperature, and the solid was washed with 10 mL of DMF and then immersed in 10 mL of DMF overnight. The solid that was a filtered material was washed three times using 10 mL of chloroform. The solid was immersed in 10 mL of chloroform all night, and after filtration, the solid was vacuum-dried at 150°C for 5 hours to obtain a metal-organic framework 3-1 as a white solid.

### [Example 3-2] to [Example 3-8]

Metal-organic frameworks 3-2 to 3-8 were obtained by performing operations similar to those of Example 3-1 except that the compounds, the metal salts, and the solvents shown in the following Table 4 were used, and the reaction was performed under the reaction conditions (temperature and heating time) shown in Table 4. The results are shown in Table 4.

**[Table 4]**

| Example | Compound number | Metal salt | Solvent | Temperature (°C) | Heating time (hr) | Properties |
|---|---|---|---|---|---|---|
| **3-1** | **1** | **Zn(NO)₂·6H₂O** | **DMF** ^{water} | **120** | **12** | White solid |
| **3-2** | **1** | **Zn(NO₃)₂·6H₂O** | **DMF^{∗1}** | **120** | **24** | Colorless powder |
| **3-3** | **1** | **Co(NO₃)₂·6H₂O** | **DMF^{∗1}** | **120** | **24** | Red solid |
| **3-4** | **1** | **Co(NO₃)₂·6H₂O** | DMF ^{water} | **120** | **24** | Black/peach/ dark brown mixture solid |
| **3-5** | **6** | **Zn(NO₃)₂·6H₂O** | **DMF^{∗1}** | **120** | **24** | White solid |
| **3-6** | **6** | **Zn(NO₃)₂·6H₂O** | **DMF/**^{water} | **120** | **24** | White solid |
| **3-7** | **10** | **Zn(NO₃)₂·6H₂O** | **DMF^{∗1}** | **120** | **24** | Colorless powder |
| **3-8** | **10** | **Zn(NO₃)₂·6H₂O** | **DMF/**^{water} | **120** | **24** | Colorless powder |

| | | | | | | |
|---|---|---|---|---|---|---|
| ***1 :DMF 5ml** | | | | | | |

### [Example 3-9]

A metal-organic framework 3-9 was obtained as red crystals by performing operations similarly to Example 3-3 except that the solid concentration in the solution (representing the concentration of each of the compound of the compound number and the metal salt described in Table 4 and isonicotinic acid in the solution (g/v)) was halved.

### [Example 3-10]

A metal-organic framework 3-10 was obtained as a black/peach/dark brown mixture by performing operations similarly to Example 3-4 except that the solid concentration in the solution (representing the concentration of each of the compound of the compound number and the metal salt described in Table 4 and isonicotinic acid in the solution (g/v)) was halved.

### [Example 4-1]

3.3 mL of DMF and 6.6 mL of DEF as solvents were added to the compound of compound number 1 (0.25 mmol), zinc nitrate hexahydrate (0.25 mmol), and 1,4-diazabicyclo[2.2.2]octane (DABCO) (0.25 mmol) as an ancillary ligand, and the mixture was heated in an oven (reaction conditions: 90°C, 24 hours). The mixture was returned to room temperature, the solid was washed three times with 10 mL of DMF, and then the solid that was a filtered material was washed three times using 10 mL of chloroform. The solid was immersed in 10 mL of chloroform all night, and after filtration, the solid was vacuum-dried at 150°C for 5 hours to obtain a metal-organic framework 4-1 as a white solid.

### [Example 4-2] to [Example 4-5]

Metal-organic frameworks 4-2 to 4-5 were obtained by performing operations similar to those of Example 4-1 except that the compounds, the ancillary ligands, and the solvents shown in the following Table 5 were used, and the reaction was performed under the reaction conditions (temperature and time) shown in Table 5. The results are shown in Table 5.

**[Table 5]**

| Example | Compound number | Ancillary ligand | Solvent | Temperature (°C) | Heating time (hr) | Properties |
|---|---|---|---|---|---|---|
| **4-1** | **1** | **DABCO** | **DMF/DEF** | **90** | **24** | White powder |
| **4-2** | **1** | **Bpy^{∗1}** | **DMF/DEF** | **90** | **24** | White powder |
| **4-3** | **1** | **DABCO** | **DMF^{∗2}** | **90** | **2 4** | White powder |
| **4-4** | **1** | **DABCO** | **DMF^{∗2}** | **120** | **24** | White powder |
| **4-5** | **2** | **DABCO** | **DMF^{∗3}** | **120** | **48. 5** | Light yellow powder |

| | | | | | | |
|---|---|---|---|---|---|---|
| **∗1 : 4, 4' - Bipyridyl** **∗ 2 : DMF 10ml** **∗ 3 : DMF 15ml** | | | | | | |

### [Example 5-1]

The compound of compound number 1 (0.5 mmol) was dissolved in 7 mL of DMF. A solution of zinc acetate dihydrate (1.27 mmol) in 8 mL of DMF was dropped thereinto. The mixture was stirred at room temperature for 2.5 hours and allowed to stand. The supernatant was removed, and the solid was immersed in 20 mL of DMF overnight. Subsequently, the supernatant was removed followed by substitution using chloroform. The solid was immersed in 20 mL of chloroform overnight, the solid was separated again, and the washing operation was repeated three times. Subsequently, the separated solid was vacuum-dried at 150°C for 5 hours to obtain a metal-organic framework 5-1 as a white solid.

### [Example 5-2] to [Example 5-4]

Metal-organic frameworks 5-2 to 5-4 were obtained by performing operations similar to those of Example 5-1 except that the compounds and the solvents shown in the following Table 6 were used. The results are shown in Table 6.

**[Table 6]**

| Example | Compound number | Solvent | Properties |
|---|---|---|---|
| **5 - 1** | **1** | **DMF** | White powder |
| **5 - 2** | **1** | **D E F** | White powder |
| **5 - 3** | **2** | **DMF** | Orange powder |
| **5 - 4** | **4** | **DMF** | White powder |

### [Example 5-5]

The compound of compound number 1 (1 mmol) was dissolved in 13 mL of DMF, and triethylamine (0.28 ml) was added. A solution of zinc acetate dihydrate (2.54 mmol) in 17 mL of DMF was dropped thereinto. The mixture was stirred at room temperature for 2.5 hours and allowed to stand. The supernatant was removed, and the solid was immersed in 20 mL of DMF overnight. Subsequently, the supernatant was removed followed by substitution using chloroform. The solid was immersed in 20 mL of chloroform overnight, the solid was separated again, and the washing operation was repeated three times. Subsequently, the separated solid was vacuum-dried at 150°C for 5 hours to obtain a metal-organic framework 5-5 as a white solid.

### [Example 5-6] to [Example 5-8]

Metal-organic frameworks 5-6 to 5-8 were obtained by performing operations similar to those of Example 5-5 except that the compounds and the solvents shown in the following Table 7 were used, and the temperature and the reaction time shown in Table 7 were set. The results are shown in Table 7.

**[Table 7]**

| Example | Compound number | Solvent | Temperature (°C) | Heating time (hr) | Properties |
|---|---|---|---|---|---|
| **5-5** | **1** | **DMF** | Room temperature | **2. 5** | White powder |
| **5-6** | **1** | **DEF** | Room temperature | **2. 5** | White powder |
| **5-7** | **2** | **DMF** | Room temperature → 120°C | **2. 5→24** | Orange powder |
| **5-8** | **4** | **DMF** | Room temperature | **3** | White powder |

### [Example 6-1]

The compound of compound number 1 (0.3 mmol), cobalt nitrate hexahydrate (0.3 mmol), 5.6 mL of DMF, and 1.4 mL of ethanol were placed in an autoclave and hermetically sealed. The mixture was heated at 100°C for 21 hours and returned to room temperature. The obtained solid was separated by centrifugation. The supernatant was removed followed by substitution using chloroform. A washing operation in which the centrifuged solid was immersed in 20 mL of chloroform overnight and centrifuged again was repeated three times. Subsequently, the centrifuged solid was vacuum-dried at 150°C for 5 hours to obtain a metal-organic framework 6-1 as a gray solid.

### [Example 6-2]

A metal-organic framework 6-2 was obtained as a pale red solid by performing operations similar to those of Example 6-1 except that the compound of compound number 6 was used instead of the compound of compound number 1.

### [Example 7-1]

The compound of compound number 3 (0.4 mmol), nickel nitrate hexahydrate (0.8 mmol), 9 mL of DMF, and 1 mL of water were heated at 100°C for 16 hours and returned to room temperature. The obtained solid was filtered, and the solid that was a filtered material was washed with DMF. The solid was immersed in 20 mL of DMF all night, the solid filtered off was washed with chloroform and immersed in 20 mL of chloroform overnight, and the solid filtered off again was vacuum-dried at 150°C for 5 hours to obtain a metal-organic framework 7-1 as a dark brown solid.

### [Example 7-2]

A metal-organic framework 7-2 was obtained as a dark brown solid by performing operations similar to those of Example 7-1 except that cobalt nitrate hexahydrate was used instead of nickel nitrate hexahydrate.

### [Example 7-3]

A metal-organic framework 7-3 was obtained as a light green solid by performing operations similar to those of Example 7-1 except that the compound of compound number 4 and THF/water (18 ml/2 ml) as the solvent were used, the reaction time was 48 hours, and solid-liquid separation was performed by centrifugation.

### [Example 8-1]

The compound of compound number 3 (0.5 mmol), magnesium nitrate hexahydrate (1.0 mmol), 7 mL of THF, 3 mL of water, and 2 mL of a 1 N NaOH aqueous solution were heated at 100°C for 24 hours and returned to room temperature. The obtained solid was filtered, and the solid that was a filtered material was washed with DMF. The solid was immersed in 20 mL of DMF all night, the solid filtered off was washed with chloroform and immersed in 20 mL of chloroform overnight, and the solid filtered off again was vacuum-dried at 150°C for 5 hours to obtain a metal-organic framework 8-1 as a dark brown solid.

### [Example 9-1]

The compound of compound number 5 (0.84 mmol), copper nitrate hemipentahydrate (1.5 mmol), 5 mL of ethanol, and 5 mL of water were stirred at room temperature for 5 minutes. The mixture was heated at 140°C for 24 hours and returned to room temperature. The reaction product was centrifuged, and the obtained solid was washed with DMF. The centrifuged solid was washed with chloroform and immersed in 20 mL of chloroform overnight, and the solid centrifuged again was vacuum-dried at 150°C for 5 hours to obtain a metal-organic framework 9-1 as a blue powder.

### [Example 10-1]

The compound of compound number 1 (118.3 mg, 0.60 mmol), zirconium tetrachloride (140.1 mg, 0.60 mmol), N,N-dimethylformamide (DMF) (8 mL), water (130 mg, 12 eq.), and acetic acid (1.803 g, 30 eq.) were placed in a screw cap vial, and ultrasonic treatment was performed. Subsequently, the screw cap vial was sealed, and the mixture was heated at 120°C for 24 hours. The mixture was cooled to room temperature, centrifuged, and decanted to obtain the solid. The operation of adding DMF to the solid and performing centrifugation and decantation was repeated three times. The solvent was changed to acetone, a similar operation was repeated three times, and the solid was washed and then immersed in acetone for 24 hours. After centrifugation and decantation, the solid was vacuum-dried at 150°C for approximately 6 hours to obtain a metal-organic framework 10-1 (159.1 mg) as an off-white powder.

### [Examples 10-2 to 10-5]

Metal-organic frameworks 10-2 to 10-5 were obtained by performing operations similar to those of Example 10-1 except that the compounds shown in the following Table 8 were used, and the reaction was performed under the reaction conditions shown in Table 8. The results are shown in Table 8.

**[Table 8]**

| Example | Compound number | Heating time (hr) | Acid | Properties |
|---|---|---|---|---|
| 10-1 | 1 | **24** | Acetic acid | Off-white powder |
| 10-2 | 9 | **24.5** | Acetic acid | White powder |
| 10-3 | 6 | **24** | Acetic acid | White powder |
| 10-4 | 2 | **24** | Acetic acid | Light yellow powder |
| 10-5 | 1 | **24** | Benzoic acid (26 eq.) | Light yellow crystals |

### [Example 10-6]

The compound of compound number 6 (112.4 mg, 0.50 mmol), cobalt nitrate hexahydrate (145.8 mg, 0.50 mmol), and isonicotinic acid (62.1 mg, 0.5 mmol) were dissolved in 5 mL of N,N-dimethylformamide (DMF), the solution was placed in a screw cap vial, and ultrasonic treatment was performed. Subsequently, the screw cap vial was sealed, and the solution was heated at 120°C for 26 hours. The solution was cooled to room temperature, centrifuged, and decanted to obtain the solid. The operation of adding DMF to the solid and performing centrifugation and decantation was repeated three times. The solvent was changed to chloroform, a similar operation was repeated three times, and the solid was washed and then immersed in chloroform for 24 hours. After centrifugation and decantation, the solid was vacuum-dried at 150°C for approximately 6 hours to obtain a metal-organic framework 10-6 (123.5 mg) as a purple powder.

### [Example 10-7]

A metal-organic framework 10-7 was obtained as a purple powder by performing operations similarly to Example 10-6 except that the reaction temperature was 90°C.

### [Example 10-8]

The compound of compound number 1 (78.8 mg, 0.40 mmol) and copper nitrate trihydrate (96.7 mg, 0.40 mmol) were dissolved in 8 mL of N,N-dimethylformamide (DMF), the solution was placed in a screw cap vial, and ultrasonic treatment was performed. Subsequently, the screw cap vial was sealed, and the solution was heated at 120°C for 24 hours. The solution was cooled to room temperature, centrifuged, and decanted to obtain the solid. The operation of adding DMF to the solid and performing centrifugation and decantation was repeated three times. The solvent was changed to chloroform, a similar operation was repeated three times, and the solid was washed and then immersed in chloroform for 24 hours. After centrifugation and decantation, the solid was vacuum-dried at 150°C for approximately 6 hours to obtain a metal-organic framework 10-8 (23.4 mg) as a dark green powder.

### [Examples 10-9 to 10-10]

Metal-organic frameworks 10-9 to 10-10 were obtained by performing operations similar to those of Example 10-8 except that the compounds shown in the following Table 9 were used, and the reaction was performed under the reaction conditions shown in Table 9. The results are shown in Table 9.

**[Table 9]**

| Example | Compound number | Properties |
|---|---|---|
| 10-8 | **1** | Dark green powder |
| 10-9 | **9** | Light green powder |
| 10-10 | **6** | Light green powder |

### [Example 10-11]

The compound of compound number 1 (78.8 mg, 0.40 mmol) and nickel nitrate hexahydrate (233.2 mg, 0.80 mmol) were dissolved in 9 mL of THF and 1 mL of water, and the solution was placed in an autoclave and heated at 100°C for 48 hours. The solution was cooled to room temperature, centrifuged, and decanted to obtain the solid. The operation of adding DMF to the solid and performing centrifugation and decantation was repeated three times. The solvent was changed to chloroform, a similar operation was repeated three times, and the solid was washed and then immersed in chloroform for 24 hours. After centrifugation and decantation, the solid was vacuum-dried at 150°C for approximately 6 hours to obtain a metal-organic framework 10-11 (47.9 mg) as a yellow-green powder.

### [Example 10-12]

A metal-organic framework 10-12 was obtained as a light green powder by performing operations similar to those of Example 10-11 except that the compound of compound number 6 was used instead of the compound of compound number 1.

### [Example 11]

### (BET Specific Surface Area Measurement and Measurement of Amount of Hydrogen Stored)

For some of the obtained metal-organic frameworks, the BET specific surface area, and the amount of hydrogen stored at temperature 77K-atmospheric pressure were measured.

The measurement of the BET specific surface area, and the amount of hydrogen stored at temperature 77K-atmospheric pressure was performed using an apparatus for measuring the amount of gas adsorbed, Tristar-II (manufactured by Micromeritics).

The BET specific surface area was calculated by the following method. Approximately 50 mg of a metal-organic framework was placed inside a glass cell. The pressure inside the glass cell was reduced to a vacuum at a temperature of 135°C, and the inside of the glass cell was dried for 6 hours. The glass cell was mounted in the apparatus for measuring the amount of gas adsorbed, and immersed in a constant temperature bath containing liquid nitrogen. The pressure of nitrogen contained in the glass cell was gradually increased. The measurement was performed until the pressure of nitrogen introduced inside the glass cell reached 1.0 × 10⁵ Pa.

The amount of hydrogen stored at a temperature of 77K and normal pressure was calculated by the following method. After the measurement of nitrogen, the gas species was changed to hydrogen, and the measurement was performed. The pressure of hydrogen contained in the glass cell was gradually increased. The measurement was performed until the pressure of hydrogen introduced inside the glass cell reached 1.0 × 10⁵ Pa.

The results of the measured BET specific surface area are shown in Table 10.

The amount of hydrogen stored at temperature 77K-atmospheric pressure measured is shown in Table 11.

**[Table 10]**

| Metal-organic framework number | BET specific surface area (m²/g) | Metal-organic framework number | BET specific surface area (m²/g) |
|---|---|---|---|
| 1-1 | 2. 18 | 3-1 | 1. 49 |
| 1-2 | 1321 | 3-2 | 0. 9 |
| 1-3 | 13. 8 | 3-3 | 493 |
| 1-4 | 19. 6 | 3-4 | 208 |
| 1-5 | 2. 2 | 3-5 | 9.21 |
| 1-6 | 9. 72 | 3-6 | 7 |
| 1-7 | 44.8 | 3-7 | 9.3 |
| 1-8 | 52.4 | 3-8 | 0.78 |
| 1-9 | 9. 44 | 3-9 | 439 |
| 1-10 | 0. 8 | 3-10 | 58 |
| 1-11 | 429 | 4-1 | 42.4 |
| 1-12 | 3 | 4-2 | 22 |
| 1-13 | 3. 1 | 4-3 | 87 |
| 1-14 | 48. 5 | 4-4 | 58 |
| 1-15 | 23 | 4-5 | 4 |
| 1-16 | 1138 | 5-1 | 88. 7 |
| 1-17 | 1093 | 5-2 | 88. 9 |
| 1-18 | 11. 6 | 5-3 | 10. 1 |
| 1-19 | 44. 1 | 5-4 | 12. 1 |
| 1-20 | 14. 1 | 5-5 | 141.9 |
| 1-21 | 7.5 | 5-6 | 91. 3 |
| 1-22 | 10 | 5-7 | 89. 1 |
| 1-23 | 16. 2 | 5-8 | 171 |
| 1-24 | 20. 7 | 6-1 | 25. 3 |
| 1-25 | 10. 3 | 6-2 | 52.3 |
| 1-26 | 25.5 | 7-1 | 118 |
| 1-27 | 11. 7 | 7-2 | 7.34 |
| 1-28 | 9 | 7-3 | 321 |
| 1-29 | 15. 7 | 8-1 | 122 |
| 1-30 | 34.6 | 9-1 | 166 |
| 1-31 | 17 | | |
| 2-1 | 175 | | |
| 2-2 | 109 | | |
| 2-3 | 176 | | |
| 2-4 | 198 | | |
| 2-5 | 236 | | |
| 2-6 | 70. 5 | | |
| 10-1 | 366 | 10-7 | 37. 5 |
| 10-2 | 350 | 10-8 | 619 |
| 10-3 | 512 | 10-9 | 22. 2 |
| 10-4 | 465 | 10-10 | 47. 3 |
| 10-5 | 72. 8 | 10-11 | 27. 1 |
| 10-6 | 45.8 | 10-12 | 92.9 |

**[Table 11]**

| Metal-organic framework number | Amount of hydrogen stored (wt%) | Metal-organic framework number | Amount of hydrogen stored (wt%) |
|---|---|---|---|
| | 7 7 K - atmospheric pressure | | 7 7 K - atmospheric pressure |
| 1-2 | 0.556 | 3-7 | 0.021 |
| 1-4 | 0.002 | 3-8 | 0.011 |
| 1-6 | 0.063 | 3-9 | 0.869 |
| 1-6 | 0.080 | 3-10 | 0.170 |
| 1-10 | 0.138 | 4-1 | 0. 314 |
| 1-11 | 0. 350 | 4-3 | 0. 248 |
| 1-12 | 0. 080 | 4-4 | 0. 154 |
| 1-13 | 0.249 | 4-5 | 0.140 |
| 1-16 | 0.511 | 5-3 | 0. 006 |
| 1-17 | 0.467 | 5-4 | 0. 274 |
| 1-18 | 0.017 | 5-5 | 0.085 |
| 1-19 | 0. 048 | 5-6 | 0.067 |
| 1-20 | 0.154 | 5-7 | 0. 082 |
| 1-21 | 0.116 | 5-8 | 0.319 |
| 1-22 | 0.191 | 6-1 | 0. 093 |
| 1-23 | 0. 143 | 6-2 | 0. 139 |
| 1-24 | 0. 041 | 7-3 | 0. 788 |
| 1-25 | 0. 041 | 8-1 | 0. 048 |
| 1-26 | 0. 085 | 9-1 | 0.285 |
| 1-27 | 0. 083 | 10-1 | 0. 508 |
| 1-28 | 0.014 | 10-2 | 0. 552 |
| 1-29 | 0. 031 | 10-3 | 0. 683 |
| 1-30 | 0. 137 | 10-4 | 0. 521 |
| 1-31 | 0.174 | 10-5 | 0. 681 |
| 2-3 | 0. 285 | 10-6 | 0.214 |
| 2-4 | 0.219 | 10-7 | 0.104 |
| 2-6 | 0. 367 | 10-8 | 1. 499 |
| 3-3 | 0. 939 | 10-9 | 0. 509 |
| 3-4 | 0. 402 | 10-10 | 0.185 |
| 3-5 | 0. 010 | 10-11 | 0.097 |
| 3-6 | 0.008 | 10-12 | 0. 161 |

### (Measurement of Amount of Carbon Dioxide Stored)

For the obtained metal-organic framework 3-3, the amount of carbon dioxide stored at temperature 273K-atmospheric pressure and temperature 298K-atmospheric pressure was measured.

The measurement of the amount of carbon dioxide stored was performed using an apparatus for measuring the amount of gas adsorbed, Tristar-II (manufactured by Micromeritics).

Approximately 50 mg of the metal-organic framework 3-3 was placed inside a glass cell. The pressure inside the glass cell was reduced to a vacuum at a temperature of 135°C, and the inside of the glass cell was dried for 6 hours. The glass cell was mounted in the apparatus for measuring the amount of gas adsorbed, and immersed in a constant temperature bath adjusted at a temperature of 273K or a temperature of 298K. The pressure of carbon dioxide contained in the glass cell was gradually increased. The measurement was performed until the pressure of carbon dioxide introduced inside the glass cell reached 1.0 × 10⁵ Pa. The results of the amount of carbon dioxide stored measured are shown in Table 12.

**[Table 12]**

| Metal-organic framework number | Amount of carbon dioxide stored (wt%) | |
|---|---|---|
| | 273K | 298K |
| 3-3 | 7. 2 | 4. 5 |

### (Heat of Adsorption of Carbon Dioxide)

From the carbon dioxide adsorption experiment at the above different temperatures, the heat of adsorption of carbon dioxide on the metal-organic framework 3-3 was calculated. When the calculation was performed using the software attached to Tristar-II, the heat of adsorption of carbon dioxide was 31KJ•mol⁻¹.

### (Carbon Dioxide Adsorption Selectivity)

For the metal-organic framework 3-3, the carbon dioxide adsorption selectivity at 298K was estimated using the ideal adsorption solution theory method (IAST method). The carbon dioxide and nitrogen adsorption isotherms at 298K for 3-3 were fitted by the dual-site Langmuir-Freundlich equation, and the selectivity was calculated therefrom based on the ideal adsorption solution theory method. As a result, it was shown that the metal-organic framework 3-3 adsorbed carbon dioxide in 39 times the amount of nitrogen at 298K.

### (X-Ray Structure Analysis)

For the metal-organic framework 1-24 obtained in Example 1-24, X-ray structure analysis was performed under the measurement conditions shown below.

### [Measurement Conditions]

One 0.01 × 0.01 × 0.01 mm colorless and transparent crystal of the metal-organic framework 1-24 obtained in Example 1-24 was put on a micromount, and a diffraction experiment was performed using a single crystal X-ray analysis apparatus (D8 VENTURE, manufactured by Bruker). The diffraction data obtained by applying X-rays having a wavelength of 0.78192 Å to the single crystal was analyzed to determine the structure. The results are shown in Table 13.

**[Table 13]**

| | |
|---|---|
| Measurement temperature/°C | -173°C |
| Crystal size/mm | 0. 01 × 0.01 × 0. 01 |
| Color of crystal | Colorless |
| Chemical formula | C₂₄H₃₄N₆O₁₃Zn₂ |
| Molecular weight | 731. 30 |
| Crystal system | Triclinic |
| Space group | *C*2/ *c* |
| a/A | 11. 6784 (10) |
| b/Å | 12.0832 (10) |
| c/Å | 14. 9387(13) |
| α/° | 106. 585 (2) |
| β/° | 95. 815 (2) |
| γ/° | 102. 129(2) |
| V/Å³ | 1945.8(3) |
| Z | 2 |
| Reflections collected | 19112 |
| Independent reflections | 5989 |

For the metal-organic framework 3-1 obtained in Example 3-1, X-ray structure analysis was performed under conditions similar to those of the measurement shown above. The results are shown in Table 14.

**[Table 14]**

| | |
|---|---|
| Measurement temperature/°C | -173°C |
| Crystal size/mm | 0.01 × 0.01 × 0.01 |
| Color of crystal | Colorless |
| Chemical formula | C₂₂H₁₅Zn₂N₄O₈ |
| Molecular weight | 594. 16 |
| Crystal system | Monoclinic |
| Space group | *P*2₁/*n* |
| a/Å | 8. 5509(9) |
| b/Å | 29.906 (3) |
| c/Å | 8.8030 (8) |
| β/° | 92.190 (6) |
| V/Å³ | 2249.5 (4) |
| Z | 4 |
| Reflections collected | 11461 |
| Independent reflections | 3767 |

For the metal-organic framework 3-4 obtained in Example 3-4, X-ray structure analysis was performed under conditions similar to those of the measurement shown above. The results are shown in Table 15.

**[Table 15]**

| | |
|---|---|
| Measurement temperature/°C | -173°C |
| Crystal size/mm | 0.01 × 0.01 × 0.03 |
| Color of crystal | Blue |
| Chemical formula | C₈₅H₂₁Co₂N₆O₉ |
| Molecular weight | 653. 33 |
| Crystal system | Monoclinic |
| Space group | *C*2/*c* |
| a/A | 37.3165(17) |
| b/A | 11.9989(5) |
| c/Å | 17.9499(9) |
| β/° | 102.058(2) |
| V/Å³ | 7859.9(6) |
| Z | 8 |
| Reflections collected | 28390 |
| Independent reflections | 7317 |

### Industrial Applicability

The gas storage material of the present invention can store a gas such as hydrogen, carbon dioxide, methane, or acetylene at a practical level. As a result, for example, the utilization of hydrogen is easier for the arrival of a hydrogen society, and carbon dioxide, which is a greenhouse gas, can be efficiently fixed.

## Claims

1. A storage material for a gas other than nitrogen, comprising a metal-organic framework wherein a multivalent metal ion is bonded to a molecule comprising an unsubstituted or substituted hydroxamic acid group and one or more sites capable of being bonded to the multivalent metal ion.

2. The storage material for a gas other than nitrogen according to claim 1, wherein the site capable of being bonded to the multivalent metal ion is a nitrogen atom in the unsubstituted or substituted hydroxamic acid group or a nitrogen-containing heterocyclic group.

3. The storage material for a gas other than nitrogen according to claim 1 or 2, wherein the molecule comprising an unsubstituted or substituted hydroxamic acid group and one or more sites capable of being bonded to the multivalent metal ion is at least one compound selected from a group of compounds of the following formulas (I) to (III): 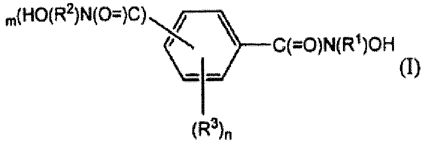 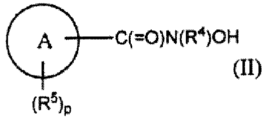 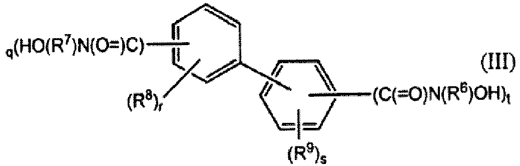 (in formulas (I) to (III),
R¹, R², R⁴, R⁶, and R⁷ each independently are a hydrogen atom, a C1-6 alkyl group, a C6-10 aryl group, a C1-6 alkylcarbonyl group, or a C6-10 arylcarbonyl group and optionally form a ring together with a carbon adjacent to a carbon of a ring to which C(=O)N(R^{u})OH (u = 1, 2, 4, 6, or 7) is bonded,
R³, R⁵, R⁸, and R⁹ each independently are a C1-6 alkyl group, a C3-8 cycloalkyl group, a C6-10 aryl group, a three-to six-membered heterocyclyl group, a C1-6 alkoxy group, a C6-10 aryloxy group, a heteroaryloxy group, a halogeno group, a C1-6 haloalkyl group, a C6-10 haloaryl group, a C1-6 haloalkoxy group, a C1-6 alkylthio group, a C6-10 arylthio group, a heteroarylthio group, a C1-6 alkylsulfinyl group, a C6-10 arylsulfinyl group, a heteroarylsulfinyl group, a C1-6 alkylsulfonyl group, a C6-10 arylsulfonyl group, a heteroarylsulfonyl group, a cyano group, a nitro group, or a group represented by NR¹¹R¹² (wherein R¹¹ and R¹² each independently are a hydrogen atom, a C1-6 alkyl group, a C6-10 aryl group, a C1-6 alkylcarbonyl group, or a C6-10 arylcarbonyl group),
m is the number of groups represented by C(=O)N(R²)OH and is 1 or 2, and when m is 2, R² is the same or different from each other,
q is the number of groups represented by C(=O)N(R⁷)OH and is 1 or 2, and when q is 2, R⁷ is the same or different from each other,
t is the number of groups represented by C(=O)N(R⁶)OH and is 1 or 2, and when t is 2, R⁶ is the same or different from each other,
n is the number of R³ and is an integer of 0 or any of 1 to 4, and when n is 2 or more, R³ is the same or different from each other,
p is the number of R⁵, and p is an integer of 0 or any of 1 to 3 when A is a five-membered ring, and is an integer of 0 or any of 1 to 4 when A is a six-membered ring, and when p is 2 or more, R⁵ is the same or different from each other,
r is the number of R⁸ and is an integer of 0 or any of 1 to 4, and when r is 2 or more, R⁸ is the same or different,
s is the number of R⁹ and is an integer of 0 or any of 1 to 4, and when s is 2 or more, R⁹ is the same or different,
provided that n + m ≤ 5, r + q ≤ 5, and s + t ≤ 5, and
in formula (II), A is a five- or six-membered aromatic heterocycle containing 1 to 4 nitrogen atoms in an integer number as a ring-constituting atom.)

4. The storage material for a gas other than nitrogen according to any one of claims 1 to 3, wherein the multivalent metal ion is an ion of at least one metal selected from the group consisting of metals of groups 2 to 13 of the periodic table of elements.

5. The storage material for a gas other than nitrogen according to any one of claims 1 to 4, wherein the multivalent metal ion is an ion of at least one metal selected from Zn, Fe, Co, Ni, Cu, Al, Zr, and Mg.

6. A method for storing a gas other than nitrogen, comprising a step of bringing a gas other than nitrogen into contact with the storage material for a gas other than nitrogen according to any one of claims 1 to 5 to adsorb or occlude the gas other than nitrogen inside the storage material for a gas other than nitrogen.

7. A storage tank for a gas other than nitrogen, filled with the storage material for a gas other than nitrogen according to any one of claims 1 to 5.

8. A metal-organic framework wherein a multivalent metal ion is bonded to a molecule comprising an unsubstituted or substituted hydroxamic acid group and one or more sites capable of being bonded to the multivalent metal ion (provided that a metal-organic framework obtained by formation of a bond between Ti⁴⁺ or Zr⁴⁺ and benzene-1,4-dicarbohydroxamic acid is excluded).

9. The metal-organic framework according to claim 8, wherein the molecule comprising an unsubstituted or substituted hydroxamic acid group and one or more sites capable of being bonded to the multivalent metal ion is at least one compound selected from a group of compounds of the following formulas (I) to (III): 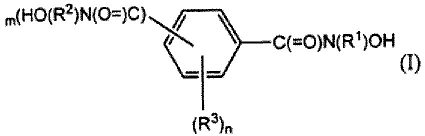 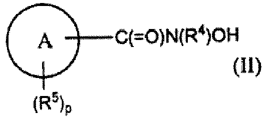 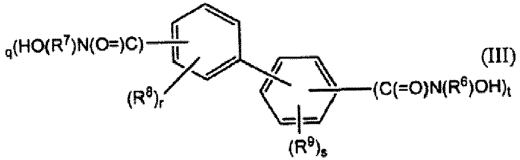 (in formulas (I) to (III),
R¹, R², R⁴, R⁶, and R⁷ each independently are a hydrogen atom, a C1-6 alkyl group, a C6-10 aryl group, a C1-6 alkylcarbonyl group, or a C6-10 arylcarbonyl group and optionally form a ring together with a carbon adjacent to a carbon of a ring to which C(=O)N(R^{u})OH (u = 1, 2, 4, 6, or 7) is bonded,
R³, R⁵, R⁸, and R⁹ each independently are a C1-6 alkyl group, a C3-8 cycloalkyl group, a C6-10 aryl group, a three-to six-membered heterocyclyl group, a C1-6 alkoxy group, a C6-10 aryloxy group, a heteroaryloxy group, a halogeno group, a C1-6 haloalkyl group, a C6-10 haloaryl group, a C1-6 haloalkoxy group, a C1-6 alkylthio group, a C6-10 arylthio group, a heteroarylthio group, a C1-6 alkylsulfinyl group, a C6-10 arylsulfinyl group, a heteroarylsulfinyl group, a C1-6 alkylsulfonyl group, a C6-10 arylsulfonyl group, a heteroarylsulfonyl group, a cyano group, a nitro group, or a group represented by NR¹¹R¹² (wherein R¹¹ and R¹² each independently are a hydrogen atom, a C1-6 alkyl group, a C6-10 aryl group, a C1-6 alkylcarbonyl group, or a C6-10 arylcarbonyl group),
m is the number of groups represented by C(=O)N(R²)OH and is 1 or 2, and when m is 2, R² is the same or different from each other,
q is the number of groups represented by C(=O)N(R⁷)OH and is 1 or 2, and when q is 2, R⁷ is the same or different from each other,
t is the number of groups represented by C(=O)N(R⁶)OH and is 1 or 2, and when t is 2, R⁶ is the same or different from each other,
n is the number of R³ and is an integer of 0 or any of 1 to 4, and when n is 2 or more, R³ is the same or different from each other,
p is the number of R⁵, and p is an integer of 0 or any of 1 to 3 when A is a five-membered ring, and is an integer of 0 or any of 1 to 4 when A is a six-membered ring, and when p is 2 or more, R⁵ is the same or different from each other,
r is the number of R⁸ and is an integer of 0 or any of 1 to 4, and when r is 2 or more, R⁸ is the same or different,
s is the number of R⁹ and is an integer of 0 or any of 1 to 4, and when s is 2 or more, R⁹ is the same or different,
provided that n + m ≤ 5, r + q ≤ 5, and s + t ≤ 5, and
in formula (II), A is a five- or six-membered aromatic heterocycle containing 1 to 4 nitrogen atoms in an integer number as a ring-constituting atom.)

10. The metal-organic framework according to claim 8 or 9, wherein the multivalent metal ion is an ion of at least one metal selected from the group consisting of metals of groups 2 to 13 of the periodic table of elements.

11. The metal-organic framework according to any one of claims 8 to 10, wherein the multivalent metal ion is an ion of at least one metal selected from Zn, Fe, Co, Ni, Cu, Al, Zr, and Mg.
